(19) Europäisches Patentamt
European Patent Office
Office européen des brevets

(11) EP 3 037 033 A1

(12) EUROPEAN PATENT APPLICATION
published in accordance with Art. 153(4) EPC

(43) Date of publication:
29.06.2016 Bulletin 2016/26

(51) Int Cl.:
A61B 5/026 (2006.01)    A61B 5/0205 (2006.01)
A61B 5/0245 (2006.01)

(21) Application number: 14850314.7

(22) Date of filing: 10.09.2014

(86) International application number:
PCT/JP2014/073977

(87) International publication number:
WO 2015/049963 (09.04.2015 Gazette 2015/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 03.10.2013 JP 2013208199

(71) Applicant: Konica Minolta, Inc.
Tokyo 100-7015 (JP)

(72) Inventors:
• TATEDA, Norihiro
  Tokyo 100-7015 (JP)
• KAMEZAWA, Hitoshi
  Tokyo 100-7015 (JP)

(74) Representative: Hoffmann Eitle
Patent- und Rechtsanwälte PartmbB
Arabellastraße 30
81925 München (DE)

(54) **BIO-INFORMATION MEASUREMENT DEVICE AND METHOD THEREFOR**

(57) Disclosed are a biological information measurement apparatus and a biological information measurement method which are configured to: emit first and second light beams, respectively, to first and second measurement positions which are different positions in a living body; receive corresponding light beams transmitted through or reflected by the living body, to acquire first and second pulse wave signals; and calculate, as biological information, a cardiac output of the living body, based on the first and second pulse wave signals. The biological information measurement apparatus and the biological information measurement method make it possible to continually monitor a change in cardiac output by a simplified apparatus.

FIG.6

CARDIAC OUTPUT CALCULATION PROCESSING

S11 ACQUISITION OF FIRST PULSE WAVE SIGNAL

S12 ACQUISITION OF SECOND PULSE WAVE SIGNAL

S13 CALCULATION OF INTER-PULSE-WAVE LAG AMOUNT

S15 CALCULATION OF PULSE RATE

S16 CALCULATION OF DIRECT-CURRENT COMPONENT

S14 CALCULATION OF PULSE WAVE VELOCITY

S17 CALCULATION OF CARDIAC OUTPUT

S18 STORAGE AND DISPLAY

TERMINATION OF PROCESSING

EP 3 037 033 A1

**Description**

Technical Field

The present information relates to a biological information measurement apparatus (bio-information measurement device) and a biological information measurement method for measuring given biological information from a time-series signal obtained from a living body.

Background Art

Heretofore, there has been known a biological information measurement apparatus for detecting biological information from a living body in a non-invasive manner. Examples of this biological information measuring apparatus include: a measurement apparatus configured to measure a pulse wave pattern and a pulse rate of a living body, called "photoelectric pulse wave meter"; and a measuring apparatus configured to measure arterial oxygen saturation, called "pulse oxymeter". A principle of the measuring apparatuses is to obtain given biological information based on a pulse wave signal which can be obtained as a signal corresponding to fluctuation due to pulsation of a biological tissue by receiving light transmitted through or reflected by a biological tissue.

One example of this biological information is cardiac output. The term "cardiac output" means an amount of blood which is pumped to an artery per minute by a heart, and can serve as an index for evaluating an ability of the heart as a pump. Therefore, a change in cardiac output is observed to follow the course of a patient, for example, with chronic heart failure.

A technique to measure the cardiac output includes a thermodilution method. This thermodilution method is configured to inject a certain amount of cold water into a right atrium by a catheter, and measure a change in temperature inside a pulmonary artery to thereby obtain the cardiac output. Further, possible alternatives include a dye dilution method configured to inject a dye in place of cold water, and measure a change in dye concentration inside a pulmonary artery.

However, in these methods, although it is possible to measure the cardiac output at a certain time point, there is difficulty in continuously measuring the cardiac output, and thereby they cannot be continually used as means for follow-up of a patient.

Therefore, a technique of continuously observing the cardiac output in a non-invasive manner is proposed, for example, in the following Patent Literature 1. A technique disclosed in the Patent Literature 1 is configured to obtain the cardiac output using a heart rate, and a pulse wave propagation time, i.e., a time required for a pulse wave to propagate from an R wave in an electrocardiogram to reach a periphery.

In this connection, in the event of a heart disease, it is effective and desirable to, before acute exacerbation, receive therapy, such as medical examination, taking of therapeutic medicines or surgery.

However, in the above technique, the pulse wave propagation time is obtained using an electrocardiogram. Thus, it is difficult to utilize the technique at home, because a subject needs to attach an electrode for electrocardiographic measurement to a given position. Thus, there is a need to make it possible to continually monitor (measure) a change in cardiac output by a simple technique usable even at home.

Citation List

Patent Literature

Patent Literature 1: JP 2005-312947A

Summary of Invention

The present invention has been made in view of the above circumstances, and an object thereof is to provide a technique capable of continually monitoring (observing, measuring) a change in cardiac output by a simple apparatus.

A biological information measurement apparatus and a biological information measurement method according to the present invention are configured to: emit first and second light beams, respectively, to first and second measurement positions which are different positions in a living body; receive receiving corresponding light beams transmitted through or reflected by the living body, to acquire first and second pulse wave signals; and calculate, as biological information, a cardiac output of the living body, based on the first and second pulse wave signals. As above, the cardiac output is obtained based on the first and second pulse wave signal. Thus, the biological information measurement apparatus and the biological information measurement method make it possible to continually monitor a change in cardiac output by a simplified apparatus.

The above and other objects, features and advantages of the present invention will be apparent from the following detailed description and the accompanying drawings.

Brief Description of Drawings

FIG. 1 is a block diagram depicting a configuration of a biological information measurement apparatus according to a first embodiment.

FIG. 2 is an external view depicting one example of the biological information measurement apparatus depicted in FIG. 1.

FIG. 3 is an external view depicting another example of the biological information measurement apparatus depicted in FIG. 1.

FIG. 4 is a diagram depicting some examples of a photoelectric pulse wave

FIG. 5 is a diagram depicting one example of a coefficient k1 in the biological information measurement apparatus depicted in FIG. 1.

FIG. 6 is a flowchart of a cardiac output calculation processing in the biological information measurement apparatus depicted in FIG. 1.

FIG. 7 is a flowchart of a cardiac output calculation processing in a first modification of the first embodiment.

FIG. 8 is a block diagram depicting a configuration of a biological information measurement apparatus according to a second modification of the first embodiment.

FIG. 9 is a flowchart of a cardiac output calculation processing in the second modification.

FIG. 10 is a diagram depicting one example of a configuration and content of a biological information record table in the second modification.

FIG. 11 is a block diagram depicting a configuration of a biological information measurement apparatus according to a second embodiment.

FIG. 12 is a diagram depicting one example of a configuration and content of a posrure determination condition table.

FIG. 13 is a diagram for explaining postures.

FIG. 14 is a flowchart of a cardiac output calculation processing in the biological information measurement apparatus depicted in FIG. 11.

FIG. 15 is a diagram depicting one example of the configuration and content of the posture determination condition table, in the biological information measurement apparatus depicted in FIG. 11.

Description of Embodiments

Based on the drawings, an embodiment of the present invention will now be described. It should be noted that elements or components assigned with the same reference sign in the figures means that they are identical, and therefore duplicated description thereof will be omitted appropriately. In this specification, when a term collectively means a plurality of identical elements or components, it is designated by a reference sign without any suffix, whereas, when the term means a specific one of the elements or components, it is designated by the reference sign with a suffix.

< First Embodiment >

If a blood flow velocity AV, a blood vessel cross-sectional area AR and a pulse rate PR are known, a cardiac output CO can be estimated (calculated) using the following formula (1), wherein the blood flow velocity AV may be substituted by a pulse wave velocity PWV:

$$CO = \alpha\, PWV \times AR \times PR \quad \text{--- (1), where } \alpha \text{ denotes a preliminarily-obtained coefficient.}$$

where $\alpha$ denotes a preliminarily-obtained coefficient.

A biological information measurement apparatus 100 according to a first embodiment of the present invention is configured to estimate the pulse wave velocity PWV, the blood vessel cross-sectional area AR and the pulse rate PR from two photoelectric pulse waves, and calculate a cardiac output using the formula (1).

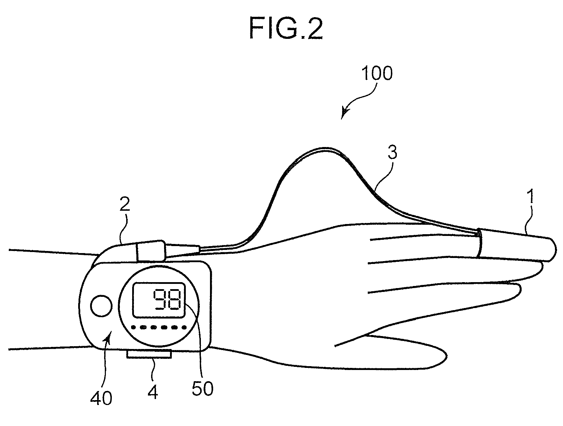

FIG. 2 depicts an external appearance (attached state) of the biological information measurement apparatus 100 according to the first embodiment. The biological information measurement apparatus 100 depicted in FIG. 2 includes a probe module 1 attached onto an end of a finger, and a measurement main module 2 attached onto a wrist, wherein the probe module 1 and the measurement main module 2 are connected together via a cable 3.

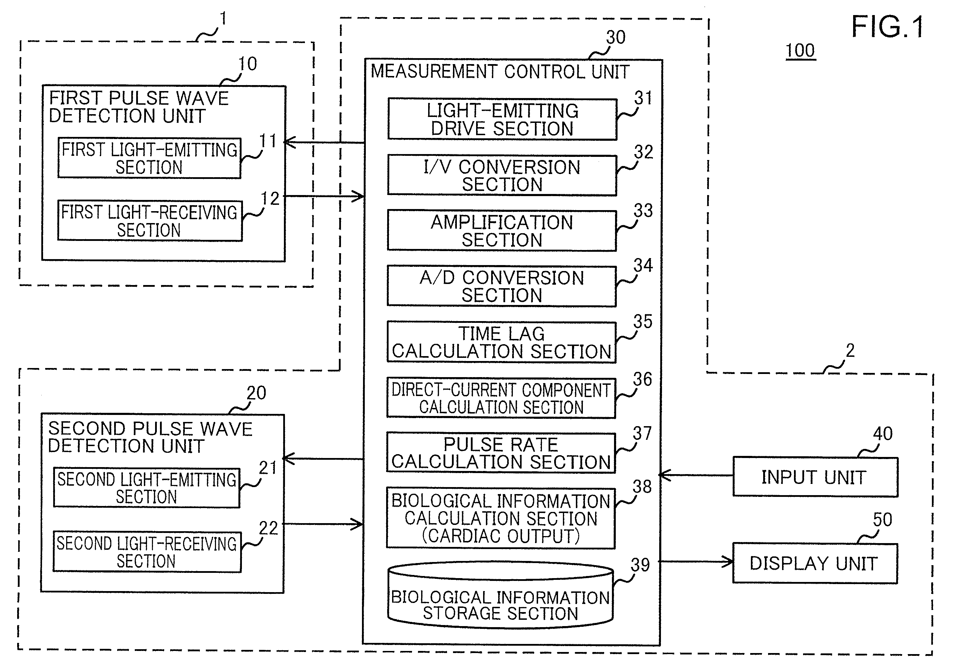

As depicted in FIG. 2, the probe module 1 is formed as a cap type, wherein it can be placed on and attached to an end of a finger such as a third finger of a left hand of a subject. The cap (probe module 1) having a circular tubular shape whose one end is closed is internally equipped with an aftermentioned first pulse wave detection unit 10 depicted in FIG. 1, wherein a pulse wave signal detected therethrough is output to the measurement main module 2 via the cable 3.

The measurement main module 2 includes a wrist band 4, and can be attached like a wristwatch by wrapping the wrist band 4 around a wrist of the left hand of the subject. The measurement main module 2 has a front surface portion provided with a display unit 50, such as a liquid crystal display, configured to display thereon biological information such as a measured cardiac output, and messages. The front surface portion of the measurement main module 2 is also provided with an input unit 40 such as a button switch. The subject can use the input unit 40 to input, into the biological information measurement apparatus 100, various instructions for start and termination of the measurement, display of a past measurement result, and others. For example, the instruction for start of the measurement is issued by an initial switch operation of the button switch, and the instruction for end of the measurement is issued by the next switch operation. Further, for example, the instruction for display of a past measurement result is issued by a long-pressing operation of the button switch (an operation of pressing down the button switch for a given time or more). The measurement main module 2 has a back surface portion equipped with an aftermentioned second pulse wave detection unit depicted in FIG. 1.

Two pulse wave signals are detected, respectively, by the probe module 1 and the measurement main module 2, in measurement areas onto which they are fixed, and biological information such as a cardiac output is calculated from the detected two pulse wave signals by a biological information calculation section comprised in the measurement main module 2, and displayed on the display unit 50.

In FIG. 2, the probe module 1 is attached onto the third finger of the left hand, and the measurement main module 2 is attached onto the wrist of the left hand. Alternatively, the two modules 1, 2 may be attached to a right hand, and the probe module 1 may be attached onto any finger other than the third finger.

The biological information measurement apparatus 100 according to the first embodiment depicted in FIG. 2 will be described below.

< Configuration >

FIG. 1 is a diagram depicting one example of a functional block configuration of the biological information measurement apparatus 100. The biological information measurement apparatus 100 includes a first pulse wave detection unit 10, a second pulse wave detection unit 20, a measurement control unit 30, the input unit 40, and the display unit 50. As delimited by the broken lines, in the biological information measurement apparatus 100, the first pulse wave detection unit 10 is incorporated in the probe module 1, and the second pulse wave detection unit 20, the measurement control unit 30, the input unit 40 and the display unit 50 are incorporated in the measurement main module 2.

The first pulse wave detection unit 10 includes a first light-emitting section 11 and a first light-receiving section 12.

The first light-emitting section 11 is composed of an IR (infrared) light-emitting element such as a light-emitting diode capable of emitting a light beam having a given first wavelength, e.g., an infrared light beam (wavelength of 850 to 950 nm). The first light-receiving section 12 is composed of an IR light-receiving element such as a silicon photodiode capable of receiving a corresponding light (infrared light) beam of the first light-emitting section 11 transmitted through or reflected by a living body, e.g., a finger.

The first light-emitting section 11 is operable to emit an infrared light beam to a biological tissue. The first light-receiving section 12 is operable to receive a corresponding infrared light beam transmitted through or reflected by the biological tissue after being emitted from the first light-emitting section 11 to the biological tissue, and subject the received infrared light beam to photoelectric conversion to thereby output, as measurement data, an electrical signal according to a light amount of the received light beam, to the measurement control unit 30. A light amount of the infrared light beam transmitted through or reflected by the biological tissue fluctuates due to pulsation of arterial blood caused by heartbeat. The first pulse wave detection unit 10 is configured to measure a fluctuation component of the infrared light beam.

The second pulse wave detection unit 20 includes a second light-emitting section 21 and a second light-receiving section 22.

The second light-emitting section 21 is composed of a G (green) light-emitting element such as a light-emitting diode capable of emitting a light beam having a given second wavelength, e.g., a green light beam (wavelength of 500 to 600 nm). The second light-receiving section 22 is composed of a G light-receiving element such as a silicon photodiode capable of receiving a corresponding light (green light) beam of the second light-emitting section 21 transmitted through or reflected by a living body, e.g., a finger.

The second light-emitting section 21 is operable to emit a green light beam to a biological tissue. The second light-receiving section 22 is operable to receive a corresponding green light beam transmitted through or reflected by the biological tissue after being emitted from the second light-emitting section 21 to the biological tissue, and subject the received green light beam to photoelectric conversion to thereby output, as measurement data, an electrical signal according to a light amount of the received light beam, to the measurement control unit 30.

The measurement control unit 30 includes a light-emitting drive section 31, an I-V conversion section 32, an amplification section 33, an A-D conversion section 34, a time lag calculation section 35, a direct-current component calculation section 36, a pulse rate calculation section 37, a biological information calculation section 38, and a biological information storage section 39. The measurement control unit 30 is configured to obtain given biological information regarding a living body as a measurement target, based on the two pieces of measurement data measured by the first pulse wave detection unit 10 and the second pulse wave detection unit 20. The measurement control unit 30 is constructed, for example, of a microcomputer having a microprocessor, a memory and a peripheral circuit thereof. The memory stores therein various programs such as a biological information calculation program for obtaining biological information based on the measurement data and a control program for controlling the entire biological information measurement apparatus 100, and various data such as the measurement data and data necessary for executing the programs, and the microprocessor such as a CPU (Central Processing Unit) is operable to execute the programs stored in the memory to thereby realize a part or an entirety of the above functional sections.

The light-emitting drive section 31 is configured to drive and control each of the first light-emitting section 11, the second light-emitting section 21, the first light-receiving section 12 and the second light-receiving section 22. For example, it is operable to perform drive control for starting (lighting-on) and stopping (lighting-off) of emission of each type of light beam, in the first light-emitting section 11 and the second light-emitting section 21, and drive control for starting and stopping of receiving of each type of light beam, in the first light-receiving section 12 and the second light-receiving section 22.

The I-V conversion section 32 is configured to convert a current indicative of each of two pieces of measurement data output, respectively, from the first light-receiving section 12 and the second light-receiving section 22, to a voltage indicative of each of the two pieces of measurement data. The I-V conversion section 32 is provided with two processing circuits each associated with a respective one of the two pieces of measurement data output from the first light-receiving section 12 and the second light-receiving section 22, and configured to process the two pieces of measurement data, respectively, by the processing circuits, in parallel. Alternatively, the I-V conversion section 32 may be provided with one measurement data processing circuit, and configured to alternately process the measurement data output from the first light-receiving section 12 and the measurement data output from the second light-receiving section 22, in a time-sharing manner. This point also apples to the amplification section 33 and the A-D conversion section 34.

The amplification section 33 is configured to amplify the voltage indicative of the measurement data output from the light-emitting drive section 31 and output the amplified voltage.

The A-D conversion section 34 is configured to convert analog data indicative of the measurement data output from the amplification section 33, to digital data indicative of the measurement data, and output the digital data. The A-D conversion section 34 is operable to sample the measurement data from the amplification section 33 at given sampling intervals (e.g., 100 Hz) to thereby output time-series measurement data. The sampling of the measurement data output from the first light-receiving section 12 and the measurement data output from the second light-receiving section 22 are performed in a synchronized manner. Alternatively, the sampling of the measurement data output from the first light-receiving section 12 and the measurement data output from the second light-receiving section 22 may be performed alternately in a time-sharing manner.

In the following description, digital data indicative of the measurement data output via the I-V conversion section 32, the amplification section 33 and the A-D conversion section 34 after being output from the first light-receiving section 12 will be appropriately referred to as "first pulse wave signal", and digital data indicative of the measurement data output via the I-V conversion section 32, the amplification section 33 and the A-D conversion section 34 after being output from the second light-receiving section 22 will be appropriately referred to as "second pulse wave signal".

The time lag calculation section 35 is configured to calculate a temporal lag amount Δt between a first time point at which a pulse of the first pulse wave signal has a given phase, and a second time point at which a pulse of the second pulse wave signal corresponding to the pulse of the first pulse wave signal has the given phase (the same phase as the given phase of the first pulse wave signal). That is, the time lag calculation section 35 is configured to calculate a temporal lag amount between pulses arising from the same heartbeat (corresponding pulses) in the first pulse wave signal and the second pulse wave signal.

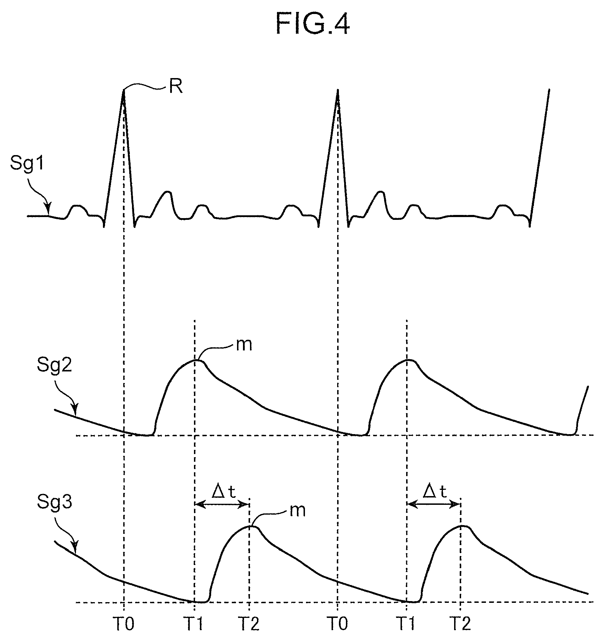

FIG. 4 depicts one example of a pulse wave signal. The upper chart of FIG. 4 depicts an electrocardiographic waveform Sg1, wherein the vertical axis represents a value of electrocardiographic data, and the horizontal axis represents a time. The mid chart of FIG. 4 depicts a second pulse wave signal Sg2 detected through the second pulse wave detection unit 20, wherein the vertical axis represents a value indicative of an amplitude of a pulse wave, and the horizontal axis represents a time. The lower chart of FIG. 4 depicts a first pulse wave signal Sg3 detected through the first pulse wave detection unit 10, wherein the vertical axis represents a value indicative of an amplitude of a pulse wave, and the horizontal axis represents a time.

The second pulse wave signal Sg2 depicted in FIG. 4 is a signal detected by the second pulse wave detection unit 20 of the measurement main module 2 attached onto the wrist. In this signal, a time from time T0 of occurrence of a peak value R of an R wave included in the electrocardiographic waveform to time T1 of occurrence of a peak value m of the second pulse wave signal Sg2 is a pulse wave propagation time required for propagation from a given position (e.g., position around the heart) to the wrist. The peak m of the second pulse wave signal Sg2 occurs, correspondingly to the R wave peak included in the electrocardiographic waveform.

Similarly, the third pulse wave signal Sg3 depicted in FIG. 4 is a signal detected by the first pulse wave detection unit 10 of the probe module 1 attached onto the end of the finger. In this signal, a time from the time T0 of occurrence of the peak value R of the R wave included in the electrocardiographic waveform to time T2 of occurrence of a peak value m of the first pulse wave signal is a pulse wave propagation time required for propagation from the given position to the end of the finger. The peak m of the first pulse wave signal Sg3 occurs, correspondingly to the R wave peak included in the electrocardiographic waveform.

The second pulse wave signal Sg2 is a signal detected at a position relatively close to the heart in terms of distance, and the first pulse wave signal Sg3 is a signal detected at a position relatively far from the heart in terms of distance.

That is, because the end of the finger is located farther away from the heart than the wrist, T1 < T2, and a time lag, specifically, a difference between the time T1 and the time T2, occurs. This lag amount corresponds to a pulse wave propagation time required for propagation from the second pulse wave detection unit 20 (wrist) to the first pulse wave detection unit 10 (end of the finger). The time lag calculation section 35 is configured to calculate this lag amount $\Delta t$ ($\Delta t$ = |T2 - T1|). In other words, the lag amount $\Delta t$ is a lag time between two time points at which respective pulses of the first and second pulse wave signals arising from the same beat have the same phase.

As above, the biological information measurement apparatus 100 may be configured to obtain a lag amount from two photoelectric pulse wave signals measured at two positions of a living body. Thus, the biological information measurement apparatus 100 may be configured to detect a pulse wave at two positions located on a pathway along which a pulse wave from a heart propagates, at respective different distances from the heart.

The direct-current component calculation section 36 is configured to subject the first pulse wave signal to filtering using a LPF (low-pass filter) to thereby calculate a direct-current component DC of the first pulse wave signal.

The pulse rate calculation section 37 is configured to calculate the pulse rate PR per minute from the first pulse wave signal. For example, the pulse rate calculation section 37 is configured to obtain a time interval between one peak value m and the next peak value m of the first pulse wave signal Sg3 in FIG. 4 (interval between T2 and T2), and derive the pulse rate from dividing one minute by the obtained time interval. Alternatively, the pulse rate calculation section 37 may be configured to obtain an average of a plurality of peal intervals, and derive the pulse rate from dividing one minute by the obtained time interval. Alternatively, the pulse rate calculation section 37 may be configured to obtain the pulse rate by measuring the number of peaks per minute.

In this embodiment, the direct-current component of the first pulse wave signal is calculated, and the pulse rate of the first pulse wave signal is calculated. Alternatively, the second pulse wave signal may be used therefor.

The biological information calculation section 38 is configured to calculate, as biological information, a cardiac output, i.e., an amount of blood which is pumped per minute by the heart. A calculation method therefor will be described the aftermentioned section < Calculation Method for Cardiac Output >.

The biological information storage section 39 is configured to store therein the biological information detected by the biological information calculation section 38. In conjunction with the storage, the biological information storage section 39 is operable to acquire time and date from a timer (not depicted) comprised in the measurement control unit 30, and store therein the biological information in an associated relation with the acquired time and date.

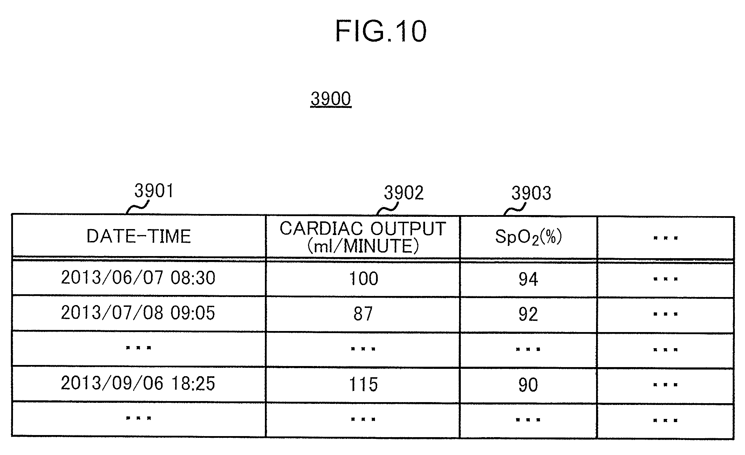

In this connection, FIG. 10 depicts one example of a configuration and content of a biological information record table 3900 stored in the biological information storage section 39. This biological information record table 3900 has a date-time field 3901, a cardiac output field 3902 and a $SpO_2$ field 3903. Every measurement, one record is registered to the biological information record table 3900.

The date-time field 3901 indicates a date-time when a measurement was performed. The cardiac output field 3902 indicates a cardiac output CO which was measured at the date-time indicated in the date-time field 3901. The $SpO_2$ field 3903 indicates a blood oxygen saturation level $SpO_2$ which was measured at the date-time indicated in the date-time field 3901.

In FIG. 10, the cardiac output CO and the blood oxygen saturation level $SpO_2$ are stored as biological information. In addition, any other biological information obtained during the course of calculating the cardiac output CO, such as the pulse rate PR, the pulse wave velocity PWV and a relative blood pressure BP may be stored.

The input unit 40 is a device for inputting, into the biological information measurement apparatus 100, an instruction for start and termination of a measurement of biological information, and others, and is composed, for example, a button located beside the display unit 50 of the measurement main module 2 depicted in FIG. 2.

The display unit 50 is a device for displaying (outputting) the measured biological information and others, and is composed, for example, of a liquid crystal display. The biological information to be displayed is not limited to the cardiac output, but may be the blood oxygen saturation level, any other biological information obtained during the course of calculating the cardiac output, such as the pulse rate, the pulse wave velocity and the blood vessel cross-sectional area, and others. All of them may be displayed on the display 50, or some of them may be selectively combined and displayed thereon.

It should be noted that a part of the functional sections of the measurement control unit 30, e.g., the I-V conversion section 32 and the amplification section 33, may be incorporated in the first pulse wave detection unit 10 and the second pulse wave detection unit 20.

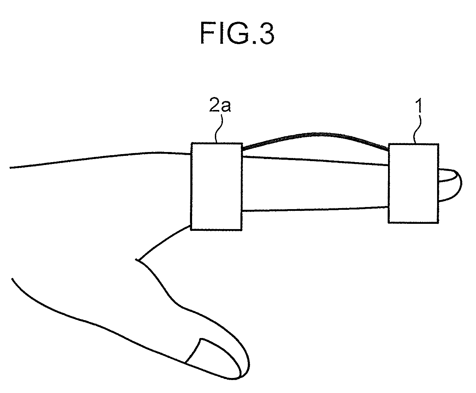

FIG. 3 depicts another example of the external appearance (attached state) of the biological information measurement apparatus 100 according to this embodiment. In the biological information measurement apparatus 100 depicted in FIG. 3, the measurement main module 2 inculdes a probe 2a equipped with the second pulse wave detection unit 20 for detecting a pulse wave signal, wherein only the probe 2a is attached onto a finger. As depicted in FIG. 3, the probe 2a of the measurement main module 2 inculdes a fixing band, and can be attached onto an index finger of a left hand of a subject by wrapping the fixing band around a region of the index finger between a base and a second knuckle thereof. In this case, for example, the measurement main module 2 provided with the measurement control unit 30, the input unit 40 and the display unit 50 is cable-connected or wirelessly connected to the probe 2a.

In FIG. 3, the probe module 1 and the probe 2a are attached onto the index finger of the left hand. Alternatively, the two elements 1, 2a may be attached to a right hand, or may be attached onto any other finger.

In FIG. 2 and FIG. 3, a pulse wave signal is detected, respectively, at two positions: a position of the end of the finger; and a position of the wrist, and at two positions: a position of the end of the finger and a position of the base of the finger. However, the two detection positions are not limited to the above positions and the above combinations. For example, the detection position may be a given position of a palm of a hand, or a given position of a back of a hand. Further, the detection position is not limited to a position of a hand, but may be a position of an end of a toe, a position of a base of a toe, a given position of an instep, a given position of a sole, or a position of an ankle. This makes it possible to measure a pulse wave signal at any position of a foot, in a situation where small fingers like newborn baby's fingers cause difficulty in measurement of a pulse wave signal.

As above, in the biological information measurement apparatus 100, it is only necessary to obtain the lag amount $\Delta t$ from two photoelectric pulse wave signals measured at two positions (two detection positions) of a living body, so that two positions located on a pathway along which a pulse wave from a heart propagates, at respective different distances from the heart are selected and used in combination.

< Calculation Method for Cardiac Output >

Next, a cardiac output calculation method to be performed by the biological information calculation section 38 will be described.

The cardiac output is calculated using the aforementioned formula (1). In this formula, the unit of the cardiac output CO is ml, the unit of the pulse wave velocity PWV is m/sec, and the unit of the blood vessel cross-sectional area AR is $cm^2$.

The pulse wave velocity PWV is calculated using the following formula (2): PWV = D /$\Delta t$ --- (2), where: $\Delta t$ denotes the inter-pulse-wave lag amount $\Delta t$ (see FIG. 4) calculated by the time lag calculation section 35; and D denotes a distance between the first pulse wave detection unit 10 and the second pulse wave detection unit 20, and, more specifically, a distance between a position where the first pulse wave detection unit 10 detects a pulse wave, and a position where the second pulse wave detection unit 20 detects a pulse wave.

In design of the biological information measurement apparatus 100, how long the distance between the first pulse wave detection unit 10 and the second pulse wave detection unit 20 (distance D) is set can be appropriately determined, considering a blood flow velocity and a sampling rate of the pulse wave.

Although the blood flow velocity varies depending on position of a living body, it is roughly in the range of 20 to 60 cm/sec. Thus, in the case where a sampling rate of measurement data output from each of the first light-receiving section 12 and the second light-receiving section 22 is 100 Hz, the lag amount $\Delta t$ can be calculated with sufficient temporal resolution, as long as the distance D is set to any value falling within the range of 10 to 20 cm. On the other had, in the case where the sampling rate is 1kHz, as long as the distance D is set to any value falling within the range of 10 to 20 cm, the lag amount $\Delta t$ can be calculated as long as the distance D is set to any value falling within the range of 1 to 2 cm.

In the biological information measurement apparatus 100, the distance D can be regulated by setting a length of the cable 3. However, it is only necessary to capture a temporal change (temporal tendency) in cardiac output in order to find a heart disease and exacerbation thereof, and therefore it is not always necessary to calculate an absolute value of the cardiac output. This means that the distance D is not necessarily required to be accurate, but may include an error, i.e., may be deviated from an actual distance.

In this embodiment, the distance D is preliminarily set. For example, the distance is set to an average distance between a wrist and an end of a third finger. Reproducibility of a measurement value can be enhanced by setting the distance D to such a fixed value. Thus, the technique of setting the distance D to a fixed value is suited to the case where monitoring is performed with a focus on fluctuation in measurement value.

In order to obtain an absolute value of the blood flow velocity, an exact value of the distance D may be input through the input unit by a measurer.

The blood vessel cross-sectional area AR can be calculated based on a direct-current component of a pulse wave signal, having a correlation with the blood vessel cross-sectional area AR, using the following formula (3): AR = f (DC) --- (3), where: AR denotes the blood vessel cross-sectional area, and DC denotes the direct-current component calculated by the direct-current component calculation section 36; and f denotes a function indicative of a correlation between AR and DC. For example, it is preliminarily obtained from a plurality of actually measured values by a statistical processing or the like.

An infrared light beam is absorbed by hemoglobin in blood. Thus, in a widening phase of a blood vessel, an amount of absorption of the infrared light beam emitted from the first pulse wave detection unit 10 by hemoglobin increases, and thereby the direct-current component decreases. Thus, by observing the direct-current component DC, a value representing a correlation with a constriction state of the blood vessel or a value representing a correlation with a relative blood pressure can be obtained. That is, the blood vessel cross-sectional area AR has a correlation with the relative blood pressure BP, and the relative blood pressure BP has a correlation with the direct-current component DC. Therefore, the blood vessel cross-sectional has a correlation with the direct-current component DC.

In this embodiment, the cardiac output CO can be simply calculated using the following formula (4): CO = α PWV × f (DC) × PR --- (4). In this way, the cardiac output can be obtained only by measuring a pulse wave signal at two positions. Through comparison with a past cardiac output stored in the biological information storage section 39, a temporal fluctuation in cardiac output can be captured. This makes it possible to detect a change in symptoms in a early stage.

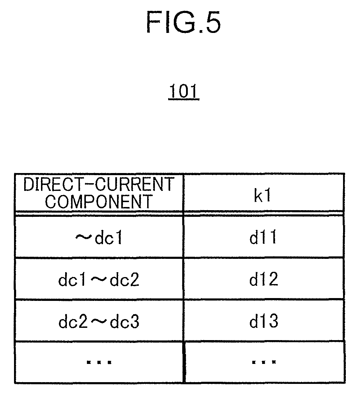

Instead of calculating the function f on a per-measurement basis, it may be preliminarily prepared so as to reduce processing (load on a CUP) during measurement. For example, as depicted in Table 101 of FIG. 5, a coefficient k1 representing a relationship with the blood vessel cross-sectional area AR, corresponding to each value of the direct-current component DC is preliminarily obtained, and stored in the measurement control unit 30. More specifically, for example, when the value of the direct-current component DC is in the range of "dc1" to "dc2", the coefficient k1 is set to a value "d12", and a value of the cardiac output CO can be simply calculated using the following formula: CO = α PWV × (k1 × DC) × PR. Values of "dc1", "dc2", "dc3" and others are appropriately set depending on a profile of the function f. For aftermentioned other functions, a similar table may be used so as to reduce processing.

< Operation >

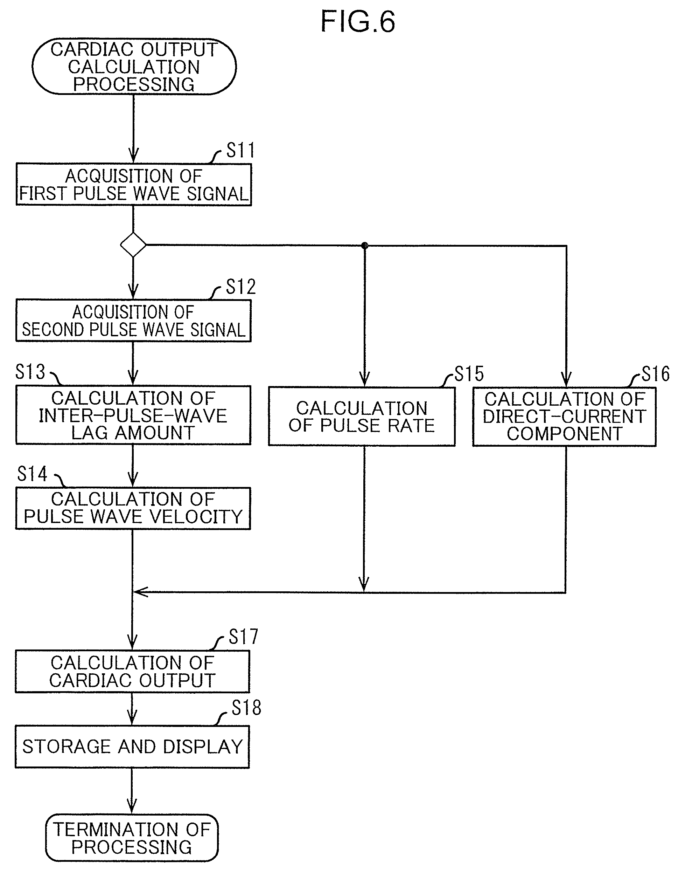

Next, an operation of the biological information measurement apparatus 100 according to this embodiment will be described. FIG. 6 is a flowchart depicting a processing for obtaining a cardiac output among biological information.

In the biological information measurement apparatus 100, upon pressing down a power switch (input unit 40) in a state in which the probe module 1 and the measurement main module 2 are attached, respectively, onto an end of a finger and a wrist as depicted in FIG. 2, a measurement of biological information for a living body as a measurement target is started.

The measurement control unit 30 instructs the light-emitting drive section 31 to drive the first pulse wave detection unit 10 and the second pulse wave detection unit 20. More specifically, the first light-emitting section 11 starts to emit a red light beam R toward the living body, and the second light-emitting section 21 starts to emit a green light beam G toward the living body. The first light-receiving section 12 starts to receive a corresponding red light beam R transmitted through the living body, and the second light-receiving section 22 starts to receive a corresponding green light beam G transmitted through the living body, whereafter they outputs, respectively, two pieces of measurement data.

The measurement control unit 30 acquires the two pieces of measurement data from the first pulse wave detection unit 10 and the second pulse wave detection unit 20, and generates (acquires) a first pulse wave signal and a second pulse wave signal, using the I-V conversion section 32, the amplification section 33 and the A-D conversion section 34 (Step S 11, Step S12).

The measurement control unit 30 outputs the first pulse wave signal to each of the direct-current component calculation section 36 and the pulse rate calculation section 37, and outputs the first pulse wave signal and the second pulse wave signal to the time lag calculation section 35.

The pulse rate calculation section 37 subjects the first pulse wave signal input from the measurement control unit 30 to processing using a bandpass filter (BPF) in which a passable frequency band (passband) is set to 0.5 to 5 Hz. The passable frequency band may be set to any range as long as pulsation can be detected from a signal passing through the filter. Then, the pulse rate calculation section 37 calculates the pulse rate PR from the filtered first pulse wave signal (Step S 15). For example, the pulse rate calculation section 37 obtains a time interval between adjacent peaks of the signal, and derives the pulse rate PR from dividing one minute by the time interval.

The direct-current component calculation section 36 subjects the first pulse wave signal input from the measurement control unit 30 to processing using a low-pass filter (LPF) in which a passable frequency band (passband) is set to several Hz or less to calculate the direct-current component DC of the first pulse wave signal (Step S16).

The time lag calculation section 35 calculates a temporal lag amount $\Delta t$ (see FIG. 4) between the first pulse wave signal and the second pulse wave signal each input from the measurement control unit 30 (Step S13).

The measurement control unit 30 operates such that the pulse rate PR calculated by the pulse rate calculation section 37, the direct-current component DC calculated by the direct-current component calculation section 36 and the lag amount $\Delta t$ calculated by the time lag calculation section 35 are output to the biological information calculation section 38.

Upon receiving inputs of the pulse rate PR, the direct-current component DC and the lag amount $\Delta t$, first of all, the biological information calculation section 38 calculates the pulse wave velocity PWV from the lag amount $\Delta t$, using the aforementioned formula (2) (Step S14). Then, the biological information calculation section 38 calculates the cardiac output CO, using the aforementioned formula (4) (Step S 17). The biological information calculation section 38 displays the calculated cardiac output CO on the display unit 50, and stores the calculated cardiac output CO in the biological information storage section 39, in associated relation with a current clock time acquired from the timer (Step S18).

< First Modification >

In the above embodiment, the cardiac output CO is obtained from the direct-current component DC. Differently, in a first modification, the relative blood pressure BP is obtained from the direct-current component DC.

The direct-current component DC and the relative blood pressure BP have a correlation therebetween, and therefore the relative blood pressure BP can be obtained using the following formula (5): BP = g (DC) --- (5), where g denotes a function indicative of a correlation between DC and BP.

Further, the relative blood pressure BP and the blood vessel cross-sectional area AR have a correlation therebetween, and therefore the blood vessel cross-sectional area AR can be obtained using the following formula (6): AR = h (BP) --- (6), where h denotes a function indicative of a correlation between BP and AR.

Thus, the cardiac output CO can be obtained using the following formula (4'): $CO = \alpha \, PWV \times (h \, (BP)) \times PR$ --- (4').

< Operation >

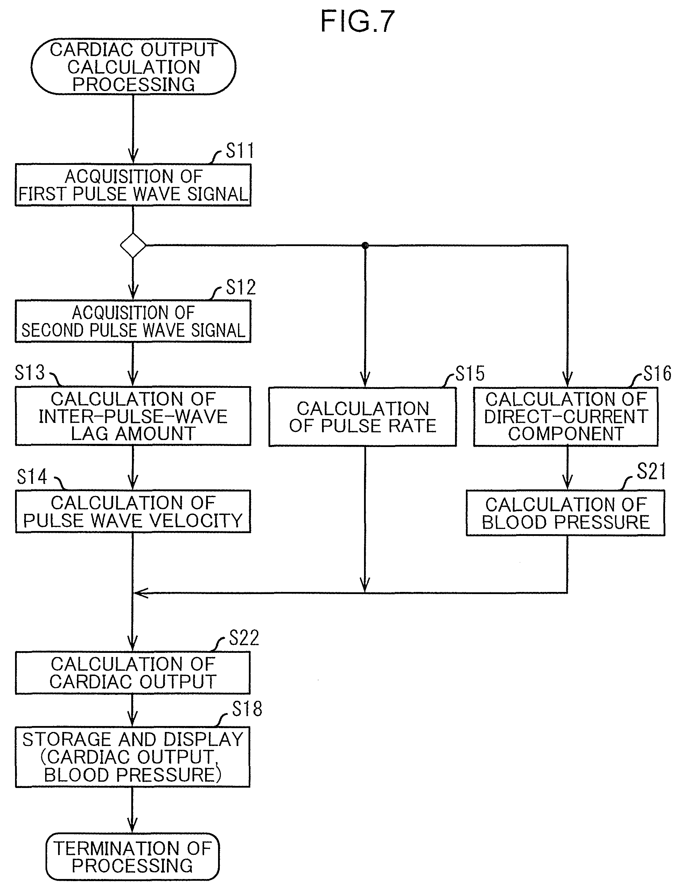

Next, with reference to a flowchart depicted in FIG. 7, an operation of a biological information measurement apparatus 100 according to the first modification will be described. A difference of a cardiac output calculation processing in the first modification from the cardiac output calculation processing in the first embodiment described with reference to FIG. 6 is only that, in a process for obtaining the blood vessel cross-sectional area AR, the relative blood pressure BP is calculated once. The following description will be made about only a difference from the flowchart depicted in FIG. 6. In the flowcharts in FIGS. 6 and 7, Steps assigned with the same numeral mean that they are identical in terms of processing.

The flowchart depicted in FIG. 7 pertaining to the first modification and the flowchart depicted in FIG. 6 pertaining to the first embodiment are different from each other in the following two points. The first point is that, after obtaining the direct-current component DC in Step S16, the relative blood pressure BP is calculated in Step S21, using the formula (5), and then Step S22 is performed. The second point is that, when the cardiac output CO is calculated in Step S22, the cardiac output CO is calculated using the relative blood pressure BP.

More specifically, upon receiving inputs of the pulse rate PR calculated by the pulse rate calculation section 37, the direct-current component DC calculated by the direct-current component calculation section 36 and the lag amount $\Delta t$ calculated by the time lag calculation section 35, first of all, the biological information calculation section 38 calculates the pulse wave velocity PWV, using the formula (2) (Step S14), and calculates the relative blood pressure BP, using the aforementioned formula (5) (Step S21). Then, the biological information calculation section 38 calculates the cardiac output CO, using the aforementioned formula (4') (Step S22). The biological information calculation section 38 displays the calculated cardiac output CO and the calculated relative blood pressure BP on the display unit 50, and stores the calculated cardiac output CO and the calculated relative blood pressure BP in the biological information storage section 39, in associated relation with a current clock time acquired from the timer (Step S18).

< Second Modification >

In the above embodiment, the cardiac output CO is calculated as biological information. Differently, in a second modification, the blood oxygen saturation level $SpO_2$ is calculated in addition to the cardiac output CO.

< Function >

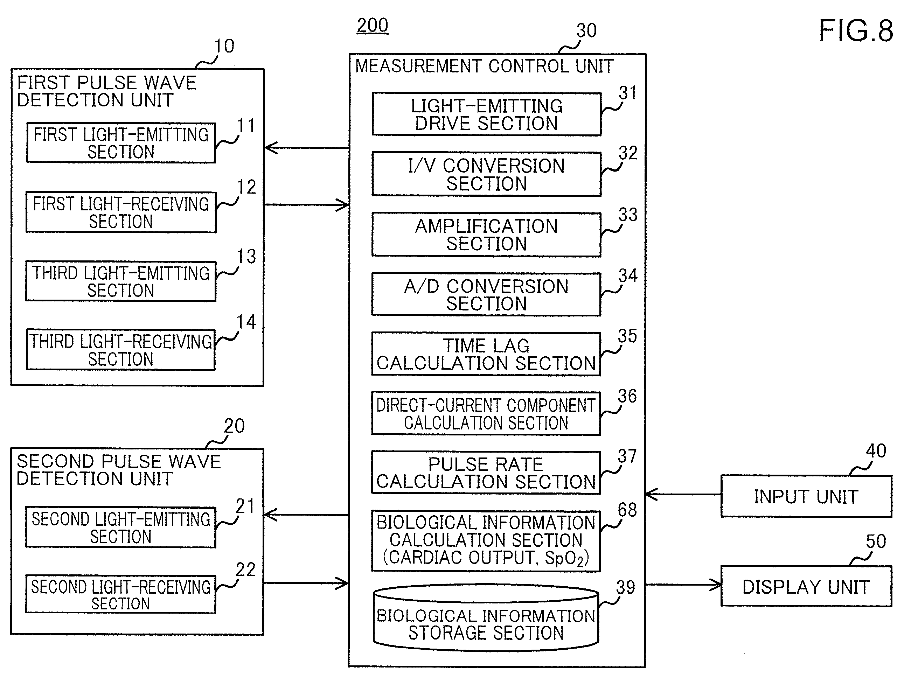

FIG. 8 is a diagram depicting one example of a functional block configuration of a biological information measurement apparatus 200 according to a second modification of the first embodiment. A difference of the biological information measurement apparatus 200 from the biological information measurement apparatus 100 according to the first embodiment described with reference to FIG. 1 is to further calculate the blood oxygen saturation level $SpO_2$. The following description will be made about only a difference from the biological information measurement apparatus 100 depicted in FIG. 1. In the biological information measurement apparatus 100 depicted in FIG. 1 and the biological information measurement apparatus 200 depicted in FIG. 8, functional blocks assigned with the same reference sign mean that they are identical in terms of function.

The biological information measurement apparatus 100 depicted in FIG. 1 and the biological information measurement apparatus 200 depicted in FIG. 8 are different from each other in the following two points. The first point is that the first pulse wave detection unit 10 further comprises a third light-emitting section 13 and a third light-receiving section 14 in order to measure a pulse wave signal necessary for calculation of the blood oxygen saturation level $SpO_2$. The second point is that the measurement control unit 30 comprises a biological information calculation section 68 in place of the biological information calculation section 38, wherein the biological information calculation section 68 is configured to calculate the blood oxygen saturation level $SpO_2$ in addition to calculating the cardiac output CO.

The third light-emitting section 13 is composed of a R (red) light-emitting element such as a light-emitting diode capable of emitting a light beam having a given third wavelength, e.g., a red light beam (wavelength of 600 to 750 nm). The third light-receiving section 14 is composed of a R light-receiving element such as a silicon photodiode capable of receiving a corresponding light (red light) beam of the third light-emitting section 13 transmitted through or reflected by a living body, e.g., a finger.

The third light-emitting section 13 is operable to emit a red light beam to a biological tissue. The third light-receiving section 14 is operable to receive a corresponding red light beam transmitted through or reflected by the biological tissue after being emitted from the third light-emitting section 13 to the biological tissue, and subject the received red light beam to photoelectric conversion to thereby output, as measurement data, an electrical signal according to a light amount of the received light beam, to the measurement control unit 30. In the following description, digital data indicative of the measurement data output via the I-V conversion section 32, the amplification section 33 and the A-D conversion section 34 of the measurement control unit 30 after being output from the third light-receiving section 14 will be referred to as "third pulse wave signal",

Heretofore, it has been known that the blood oxygen saturation level $SpO_2$ can be calculated based on measurement data acquired by emitting a plurality of light beams having respective different wavelengths, to a living body and receiving corresponding light beams transmitted through or reflected by the living body.

The blood oxygen saturation level is defined as a rate of oxygenated hemoglobin in blood. Hemoglobin has an optical property that when it is oxidized and transformed into oxyhemoglobin, absorption of red light is reduced and absorption of infrared light is increased, and on the other hand when it is reduced and returned to hemoglobin, the absorption of red light is increased and the absorption of infrared light is reduced. The blood oxygen saturation level is obtained by utilizing the difference in absorption properties of hemoglobin and oxyhemoglobin with respect to red light and infrared light.

More specifically, the blood oxygen saturation level $SpO_2$ is obtained based on time-series data about an infrared DC/AC ratio, i.e., a ratio of a direct-current component to an alternate-current component of an intensity of a transmitted or reflected light beam of an infrared light beam IR, and time-series data about a red DC/AC ratio, i.e., a ratio of a direct-current component to an alternate-current component of an intensity of a transmitted or reflected light beam of a red light beam R (see, for example, JP 4613261B).

In FIG. 8, the first light-emitting section 11 and the first light-receiving section 12 for measuring the first pulse wave signal, and the third light-emitting section 13 and the third light-receiving section 14 for measuring the third pulse wave signal are comprised in the first pulse wave detection unit 10. Alternatively, for example, the third light-emitting section 13 and the third light-receiving section 14 may be provided as a third pulse wave detection unit, separately from the first pulse wave detection unit 10.

Further, the first light-receiving section 12 and the third light-receiving section 14 may be composed of one dual-purpose light-receiving element having sensitivity to respective different wavelength bands of the two light beams to be emitted, respectively, from the first light-emitting section 11 and the third light-emitting section 13. In this case, for example, the measurement control unit 30 may be configured to control the emission of the infrared light beam IR in the first light-emitting section 11 and the emission of the red light beam R in the third light-emitting section 13 in such a manner as to be alternately performed in a time-sharing manner, to generate the first pulse wave signal and the third pulse wave signal from measurement data output from the dual-purpose light-receiving element in respective time zones.

< Operation >

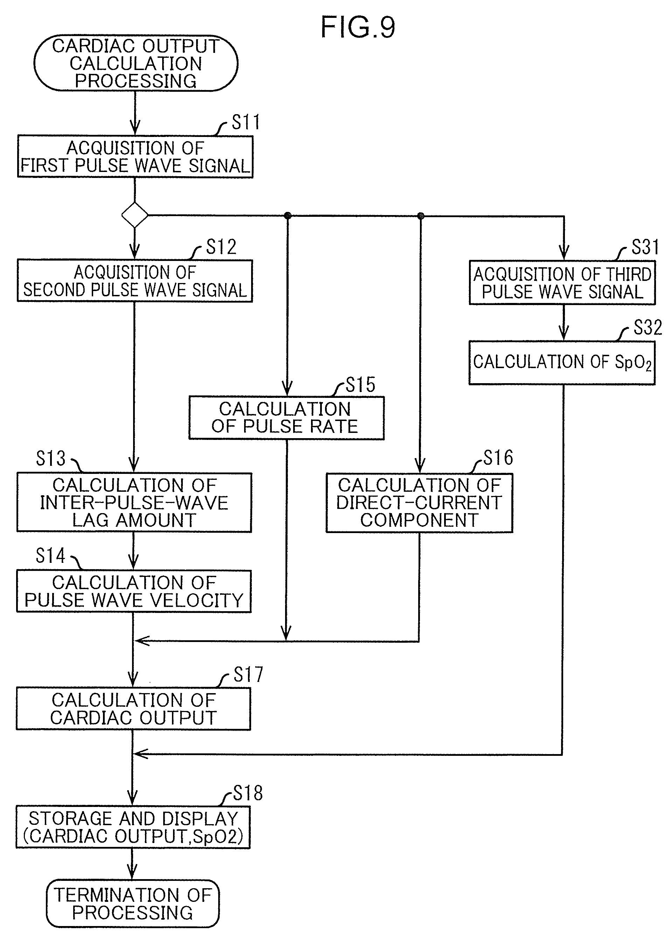

Next, with reference to the flowchart depicted in FIG. 9, an operation of the biological information measurement apparatus 200 according to the second modification will be described. A difference of a cardiac output calculation processing in the second modification from the cardiac output calculation processing in the first embodiment described with reference to FIG. 6 is to further calculate the blood oxygen saturation level $SpO_2$, as mentioned above. The following description will be made about only a difference from the flowchart depicted in FIG. 6. In the flowcharts in FIGS. 6 and 9, Steps assigned with the same numeral mean that they are identical in terms of processing.

A difference between the flowchart depicted in FIG. 9 pertaining to the second modification and the flowchart depicted in FIG. 6 pertaining to the first embodiment is that, in Step S31, the third pulse wave signal is acquired, and, in subsequent Step S32, the blood oxygen saturation level $SpO_2$ is calculated by the biological information calculation section 68.

More specifically, the measurement control unit 30 acquires red light beam R-related measurement data and infrared light beam IR-related measurement data from the first pulse wave detection unit 10, and measurement data from the second pulse wave detection unit 20, and generates (acquires) a first pulse wave signal, a second pulse wave signal and a third pulse wave signal, using the I-V conversion section 32, the amplification section 33 and the A-D conversion section 34 (Step S11, Step S12, step S31).

The measurement control unit 30 outputs the first pulse wave signal to each of the direct-current component calculation section 36 and the pulse rate calculation section 37, and outputs a set of the first pulse wave signal and the second pulse wave signal and a set of the first pulse wave signal and the third pulse wave signal, respectively, to the time lag calculation section 35 and the biological information calculation section 68.

Upon receiving inputs of the first pulse wave signal and the third pulse wave signal, the biological information calculation section 68 calculates the blood oxygen saturation level $SpO_2$, as mentioned above (Step S32).

The measurement control unit 30 operates such that the pulse rate PR calculated by the pulse rate calculation section 37, the direct-current component DC calculated by the direct-current component calculation section 36 and the lag amount $\Delta t$ calculated by the time lag calculation section 35 are output to the biological information calculation section 38. Upon receiving inputs of the pulse rate PR, the direct-current component DC and the lag amount $\Delta t$, the biological information calculation section 68 calculates the cardiac amount CO, using the aforementioned formula (4) (Step S 17).

The biological information calculation section 68 displays the calculated cardiac output CO and the calculated blood oxygen saturation level $SpO_2$ on the display unit 50, and stores the calculated cardiac output CO and the calculated blood oxygen saturation level $SpO_2$ in the biological information storage section 39, in associated relation with a current clock time acquired from the timer (Step S 18; see FIG. 10).

< Second Embodiment >

In the biological information measurement apparatus 100 according to the first embodiment, the probe module 1 is attached to the end of the finger of the left hand, and the measurement main module 2 is attached to the wrist of the left hand, as depicted in FIG. 2. A subject can move his/her hand having the biological information measurement apparatus 100 attached thereto, i.e., the measurement is not always performed in the same posture (orientation of his/her arm), e.g., in a posture where the arm is horizontally placed on a desk or the like, each time.

For example, an amount of blood flowing through a blood vessel changes between a posture where the arm is oriented upwardly and a posture where the arm is oriented downwardly. That is, the direct-current component DC also changes. This is likely to cause difficulty in accurately detecting a fluctuation in the cardiac output CO. Further, this is likely to cause difficulty in accurately calculating a value of the cardiac output CO.

Therefore, in the second embodiment, a posture of a biological information measurement apparatus 300 in an attached state is determined (estimated), the direct-current component DC is corrected depending on the posture, using the following formula (7) to provide enhanced measurement accuracy: DC2 = p (DC, X, Y, Z) --- (7), where: p denotes a DC correction function preliminarily obtained on a per-posture basis; DC denotes a direct-current component calculated by a direct-current component calculation section 36; DC2 denotes a direct-current component after correction; and X, Y and Z denote respective axial components of three axes (x, y, z), output from an aftermentioned acceleration sensor 71.

< Function >

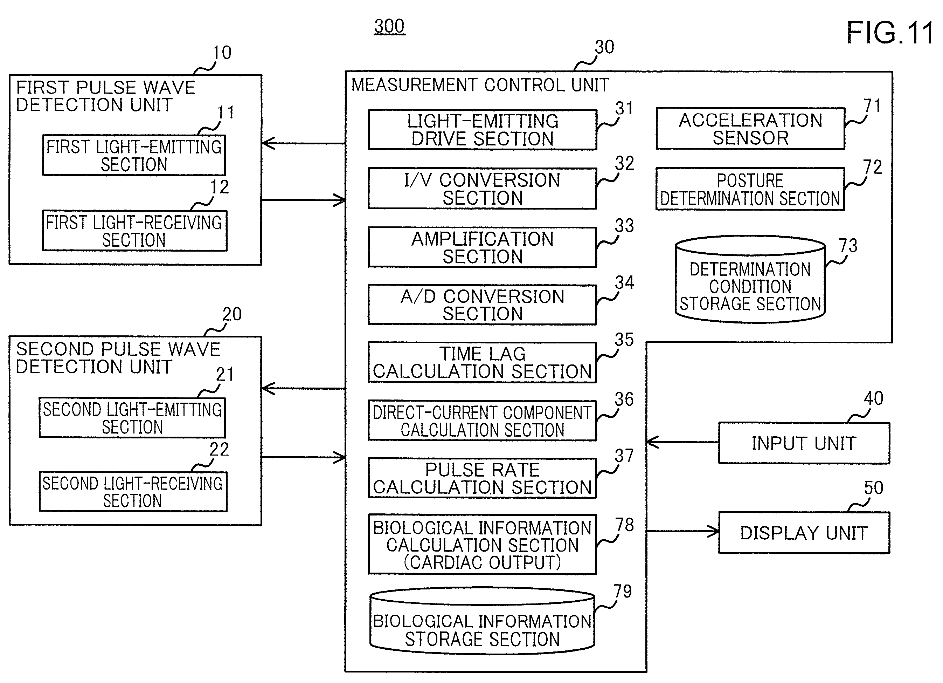

FIG. 11 is a diagram depicting one example of a functional block configuration of the biological information measurement apparatus 300 according to the second embodiment. In FIG. 11, the biological information measurement apparatus 300 comprises a first pulse wave detection unit 10, a second pulse wave detection unit 20, a measurement control unit 30, an input unit 40, and a display unit 50. The measurement control unit 30 comprises a light-emitting drive section 31, an I-V conversion section 32, an amplification section 33, an A-D conversion section 34, a time lag calculation section 35, a direct-current component calculation section 36, a pulse rate calculation section 37, an acceleration sensor 71, a posture determination section 72, a determination condition storage section 73, a biological information calculation section 78, and a biological information storage section 79. In the biological information measurement apparatus 100 depicted in FIG. 1 and the biological information measurement apparatus 300 depicted in FIG. 11, functional blocks assigned with the same reference sign mean that they are identical in terms of function.

The acceleration sensor 71 is a so-called tri-axial acceleration sensor capable of outputting a gravitational acceleration detected as respective axial components of three axes (x, y, z) of a coordinate system inside the acceleration sensor 71. From output tri-axial gravitational acceleration components, it is possible to recognize how much a measurement main module 2 incorporating the acceleration sensor 71 is inclined with respect to the ground.

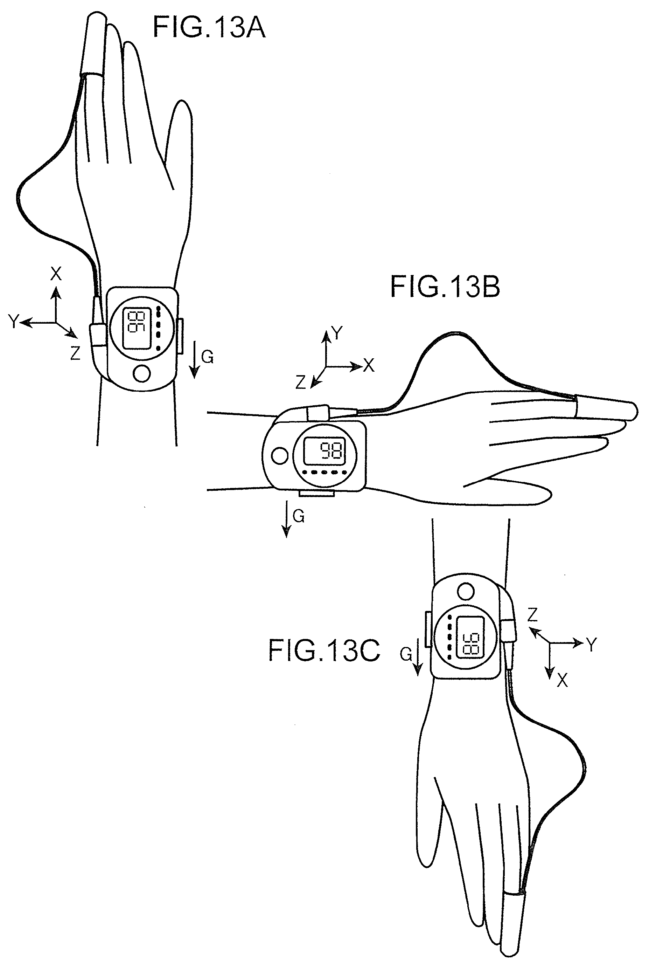

The posture determination section 72 is configured to determine an inclination of the measurement main module 2, from the gravitational acceleration components output from the acceleration sensor 71, to estimate a posture (orientation of an arm) of a subject. In this embodiment, three postures as depicted in FIG. 13 are assumed. FIG. 13A is a diagram depicting a posture where an arm having the biological information measurement apparatus 300 attached thereto is oriented upwardly. FIG. 13B is a diagram depicting a posture where the arm is oriented horizontally, and FIG. 13C is a diagram depicting a posture where the arm is oriented downwardly. The coordinate axes in each of FIGS. 13A, 13B and 13C denote the coordinate system inside the acceleration sensor 71.

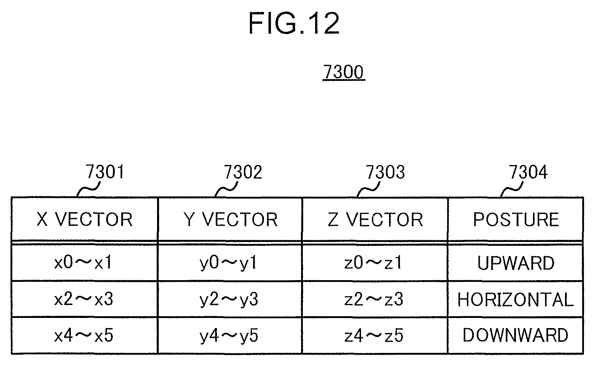

The posture determination section 72 is operable to determine the posture of the subject by referring to a posture determination condition table 7300 preliminarily stored in the determination condition storage section 73. More specifically, for example, the determination condition storage section 73 preliminarily stores therein a posture determination condition table 7300. FIG. 12 depicts one example of a configuration and content of the posture determination condition table 7300.

The posture determination condition table 7300 has an X vector field 7301, a Y vector field 7302, a Z vector field 7303 and a posture field 7304. The X vector field 7301, the Y vector field 7302 and the Z vector field 7303 present, respectively, a range of the x-axial gravitational cancellation component, a range of the y-axial gravitational cancellation component and a range of the z-axial gravitational cancellation component. The posture field 7304 presents one of the postures of the subject (determination result) when the tri-axial gravitational cancellation components output from the acceleration sensor 71 fall within the ranges presented in the X vector field 7301 to the Z vector field 7303. "Upward", "Horizontal" and "Downward" means, respectively, the posture depicted in FIG. 13A, the posture depicted in FIG. 13B and the posture depicted in FIG. 13C. For example, when the x-axial gravitational cancellation component, the y-axial gravitational cancellation component and the z-axial gravitational cancellation component each output from the acceleration sensor 71 are, respectively, in the range of "x2 to x3", in the range of "y2 to y3" and in the range of "z2 to z3", the posture determination section 72 determines that the posture of the subject is "Horizontal".

It should be noted that, although the subject's posture in the second embodiment is assumed to be three postures: a posture where the arm is oriented upwardly; a posture where the arm is oriented horizontally; and a posture where the arm is oriented downwardly, the subject's posture is not limited thereto, but may be two postures: the posture where the arm is oriented upwardly; and the posture where the arm is oriented downwardly.

< Operation >

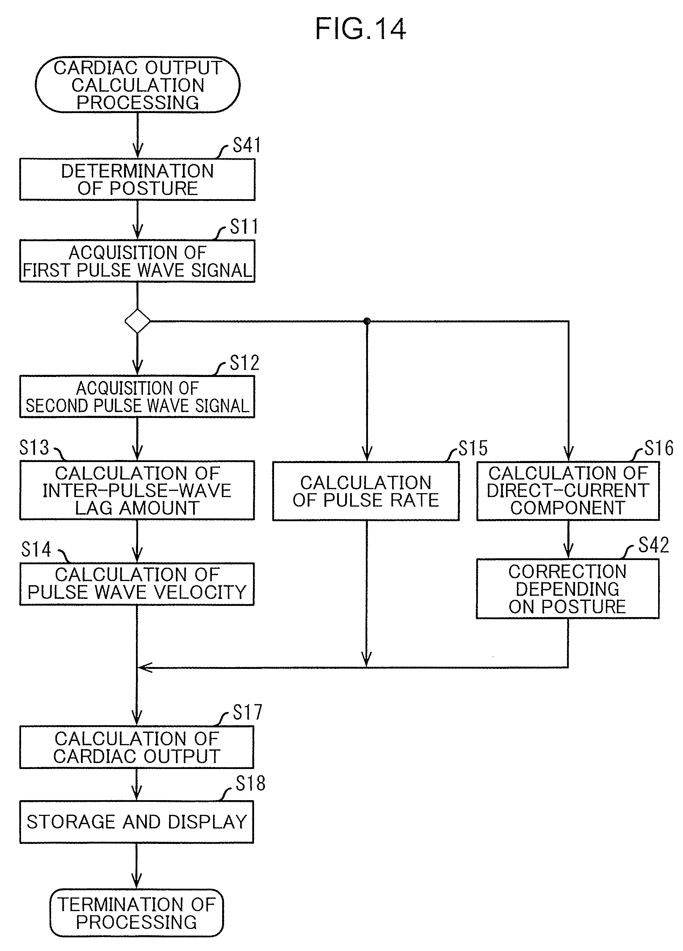

Next, with reference to the flowchart depicted in FIG. 14, an operation of the biological information measurement apparatus 300 according to the second embodiment will be described. A difference of a cardiac output calculation processing in the second embodiment from the cardiac output calculation processing in the first embodiment described with reference to FIG. 6 is to further correct the direct-current component DC depending on the posture of the subject, as mentioned above. The following description will be made about only a difference from the flowchart depicted in FIG. 6. In the flowcharts in FIGS. 6 and 14, Steps assigned with the same numeral mean that they are identical in terms of processing.

A difference between the flowchart depicted in FIG. 14 pertaining to the second embodiment and the flowchart depicted in FIG. 6 pertaining to the first embodiment is that, in Step S41, the posture (orientation of the arm) of the subject is determined, and, in Step S42, the direct-current component DC calculated by the direct-current component calculation section 36 is corrected depending on the determined posture (orientation of the arm).

More specifically, in the biological information measurement apparatus 300, upon pressing down a power switch (input unit 40) in a state in which a probe module 1 and the measurement main module 2 are attached, respectively, onto an end of a finger and a wrist as depicted in FIG. 2, a measurement of biological information for a living body as a measurement target is started.

First of all, the measurement control unit 30 operates to activate the acceleration sensor 71 and, after acquiring tri-axial gravitational acceleration components output from the acceleration sensor 71, output the acquired tri-axial gravitational acceleration components to the posture determination section 72.

Upon receiving an input of the tri-axial gravitational acceleration components, the posture determination section 72 refers to the posture determination condition table 7300 stored in the determination condition storage section 73 to determine the posture as mentioned above, and stores the determined posture and the output values (tri-axial gravitational acceleration components) from the acceleration sensor 71, in a working area (working memory) (Step S41).

Then, the measurement control unit 30 instructs the light-emitting drive section 31 to drive the first pulse wave detection unit 10 and the second pulse wave detection unit 20 to thereby generate (acquire) a first pulse wave signal and a second pulse wave signal (Step S11, Step S 12).

The measurement control unit 30 outputs the first pulse wave signal to each of the direct-current component calculation section 36 and the pulse rate calculation section 37, and outputs the first pulse wave signal and the second pulse wave signal to the time lag calculation section 35.

The pulse rate calculation section 37 subjects the first pulse wave signal input from the measurement control unit 30 to processing using the aforementioned BPF to thereby calculate the pulse rate PR from the filtered first pulse wave signal (Step S15).

The direct-current component calculation section 36 subjects the first pulse wave signal input from the measurement control unit 30 to processing using the aftermentioned LPF to thereby calculate the direct-current component DC (Step S16).

The time lag calculation section 35 calculates a temporal lag amount $\Delta t$ (see FIG. 4) between the first pulse wave signal and the second pulse wave signal each input from the measurement control unit 30 (Step S13).

The measurement control unit 30 operates such that the pulse rate PR calculated by the pulse rate calculation section 37, the direct-current component DC calculated by the direct-current component calculation section 36, the lag amount $\Delta t$ calculated by the time lag calculation section 35 and the output values of the acceleration sensor 71 stored in the working area in Step S41 are output to the biological information calculation section 78.

Upon receiving inputs of the pulse rate PR, the direct-current component DC, the lag amount $\Delta t$ and the output values of the acceleration sensor 71, first of all, the biological information calculation section 78 calculates the pulse wave velocity PWV from the lag amount $\Delta t$, using the aforementioned formula (2) (Step S 14). Then, the biological information calculation section 78 calculates a corrected direct-current component DC, using the aforementioned formula (7) (Step S42).

Subsequently, based on the corrected direct-current component DC, the pulse rate PR and the pulse wave velocity PWV, the biological information calculation section 78 calculates the cardiac output CO, using the aforementioned formula (4) (Step S 17). The biological information calculation section 78 displays the calculated cardiac output CO on the display unit 50, and stores the calculated cardiac output CO in the biological information storage section 79, in associated relation with a current clock time acquired from the timer and the posture (orientation of the arm) stored in the working area (Step S18).

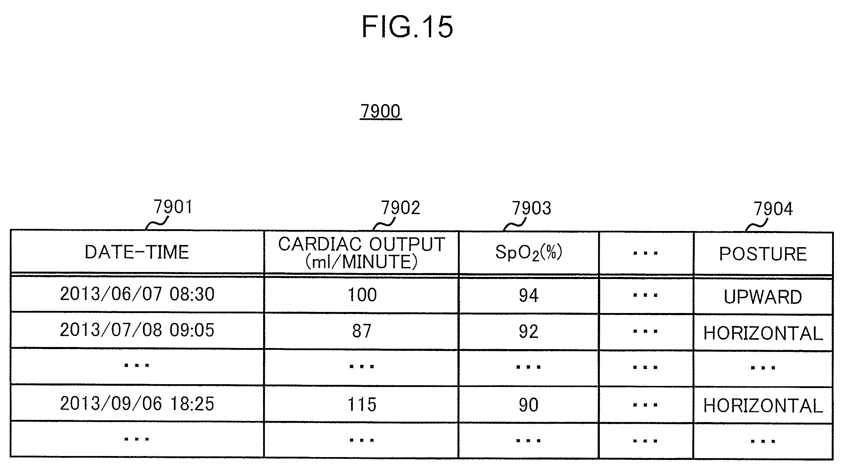

FIG. 15 depicts one example of the configuration and content of a posture determination condition table 7900 stored in the biological information storage section 79. The biological information record table 7900 has a date-time field 7901, a cardiac output field 7902, a SpO$_2$ field 7903 and a posture field 7904. The posture determination condition table 7900 is prepared by adding the posture field 7904 to the posture determination condition table 3900 depicted in FIG. 10. That is, every measurement, a posture determined by the posture determination section 72 in Step S41 is set in the posture field 7904. The date-time field 7901, the cardiac output field 7902 and the SpO$_2$ field 7903 are the same as the date-time field 3901, the cardiac output field 3902 and the SpO$_2$ field 3903 in the biological information record table 3900, respectively.

In the second embodiment, after determining the posture by the posture determination section 72, biological information is measured, irrespective of what kind of type the determined posture is, and the determined posture and the measured biological information are stored in the biological information storage section 79 in associated relation with each other. Alternatively, the apparatus may be configured such that, after determining the posture by the posture determination section 72, biological information is measured only if the determined posture is a predetermined specific posture. For example, the apparatus may be configured such that, only when the posture of the subject determined in Step S41 is "Horizontal", biological information is measured, and when the posture is other posture, a message for prompting the subject to orient the arm horizontally is displayed before measurement of biological information. More specifically, only when x-axial, y-axial and z-axial gravitational acceleration components output from the acceleration sensor 71 fall, respectively, within the range set in the X vector field 7301, the range set in the Y vector field 7302 and the range set in the Z vector field 7303, on the row where the posture field 7304 is set as "Horizontal", the routine proceeds from Step S41 to Step S11 to start to measure pulse waves.

In the second embodiment, the direct-current component DC is corrected. Alternatively, the apparatus may be configured to correct the blood vessel cross-sectional area AR, or to, in the case where the relative blood pressure BP is obtained to calculate the cardiac output CO, as in the first modification of the first embodiment, correct the relative blood pressure BP. In this case, a function (coefficient) for correcting the blood vessel cross-sectional area AR or a function (coefficient) for correcting the relative blood pressure BP is preliminarily obtained depending on the posture of the subject (inclination of the biological information measurement apparatus 100).

In the above embodiments, the first light-emitting section 11 of the first pulse wave detection unit 10 is configured to emit an infrared light beam IP, and the second light-emitting section 21 of the second pulse wave detection unit 20 is configured to emit a green light beam G. However, the present invention is not limited thereto. For example, each of the first and second light-emitting sections 11, 21 may be configured to emit any one of a red light beam R, an infrared light beam IR and a green light beam G. In this case, the first and second light-emitting sections 11, 21 may be configured to emit respective light beams having different colors or may be configured to emit respective light beams having the same color. Further, a light beam to be emitted from each of the first and second light-emitting sections 11, 21 is not limited the above light beams, but may be a light beam having any other color such as white. In other words, it may be any light beam as long as a pulse wave signal can be acquired therefrom. Each of the first pulse wave detection unit 10 and the second pulse wave detection unit 20 may be either one of a reflection type and a transmission type. However, the reflection type is preferably used when a pulse wave signal is measured at a wrist, and it is desirable to use an emission light beam having a suitable wavelength for each of the reflection type and the transmission type. It should be noted that, in the case where the blood oxygen saturation level $SpO_2$ is obtained as in the second modification of the first embodiment, the first light-emitting section 11 and the third light-emitting section 13 of the first pulse wave detection unit 10 are required to emit light beams capable of allowing the blood oxygen saturation level $SpO_2$ to be obtained, e.g., a red light beam R and an infrared light beam IR.

This specification discloses techniques having various aspects. Among them, major techniques will be outlined below.

According to one aspect, there is provided a biological information measurement apparatus which includes: a first pulse wave signal acquisition unit configured to emit a first light beam to a first measurement position of a living body, and receive a corresponding light beam transmitted through or reflected by the living body, to acquire a first pulse wave signal; a second pulse wave signal acquisition unit configured to emit a second light beam to a second measurement position of the living body different from the first measurement position, and receive a corresponding light beam transmitted through or reflected by the living body, to acquire a second pulse wave signal; a time lag calculation section configured to calculate a temporal lag amount between a first time point at which the first pulse wave signal has a given phase, and a second time point at which the second pulse wave signal corresponding to the first pulse wave signal has the given phase; a direct-current component calculation section configured to calculate a direct-current component, based on either one of the first pulse wave signal and the second pulse wave signal; a pulse rate calculation section configured to calculate a pulse rate of the living body, base on either one of the first pulse wave signal and the second pulse wave signal; and a biological information calculation section configured to calculate, as biological information, a cardiac output of the living body, based on the lag amount, the direct-current component and the pulse rate.

According to another aspect, there is provided a biological information measurement method for use with a biological information measurement apparatus for, based on a first pulse wave signal and a second pulse wave signal obtained, respectively, from a first measurement position and a second measurement position which are different positions in a living body, to measure biological information of the living body. The biological information measurement method comprises: a first pulse wave signal acquisition step of emitting a first light beam to the first measurement position, and receiving a corresponding light beam transmitted through or reflected by the living body, to acquire the first pulse wave signal; a second pulse wave signal acquisition step of emitting a second light beam to the second measurement position, and receiving a corresponding light beam transmitted through or reflected by the living body, to acquire the second pulse wave signal; a time lag calculation step of calculating a temporal lag amount between a first time point at which the first pulse wave signal has a given phase, and a second time point at which the second pulse wave signal corresponding to the first pulse wave signal has the given phase; a direct-current component calculation step of calculating a direct-current component, based on either one of the first pulse wave signal and the second pulse wave signal; a pulse rate calculation step of calculating a pulse rate of the living body, base on either one of the first pulse wave signal and the second pulse wave signal; and a biological information calculation step of calculating, as biological information, a cardiac output of the living body, based on the lag amount, the direct-current component and the pulse rate.

In the above biological information measurement apparatus and biological information measurement method, the cardiac output can be calculated from pulse wave signals measured at two different positions, i.e., the first measurement position and the second measurement position, so that it becomes possible to measure the cardiac output at home without visiting a hospital. In the above biological information measurement apparatus and biological information measurement method, it is only necessary to measure a pulse wave, so that a change in the cardiac output can be continually monitored by attaching to a subject a device used in the biological information measurement apparatus to detect a pulse wave. For example, generally, a probe module used in a so-called pulse oxymeter or the like to measure a photoelectric pulse wave can be relatively easily attached into an end of a finger of a hand. Thus, the probe or the like can be used as the device for detecting a pulse wave, to allow a measurement of a pulse wave signal to be continually performed at home.

That is, the above biological information measurement apparatus and biological information measurement method make it possible to continually evaluate a cardiac function at home, based on the cardiac output which is an objective, direct index in diagnosis of disease, instead of a non-objective, indirect index in diagnosis of disease, such as subjective symptom (respiratory failure or the like), blood pressure or pulsation, so that it becomes possible to increase a possibility of capturing the occurrence of heart failure or a sign of recurrence. Thus, the above biological information measurement apparatus and biological information measurement method make it possible to reduce a problem of missing a timing of receiving therapy (such as medical examination, taking of therapeutic medicines or surgery). The above biological information measurement apparatus and biological information measurement method make it possible to simply evaluate an cardiac function, so that it becomes possible to timely evaluate the cardiac function to find disease in an early stage, and find a sign of disease, thereby leading to prevention of disease.

Preferable, in the above biological information measurement apparatus, the first measurement position and the second measurement position are located on a pathway along which a pulse wave from a heart propagates, at respective different distances from the heart.

In the biological information measurement apparatus having this feature, the two positions for measuring the pulse wave signals thereat may be two positions located at respective different propagation distances from the heart. Thus, this biological information measurement apparatus can measure pulse wave signals to measure the cardiac output, at appropriate positions depending on conditions of disease or a subject's body.

Preferably, in the above biological information measurement apparatus, the first measurement position and the second measurement position are two positions of the living body selected from the group consisting of a position of an end of a finger, a position of a base of the finger, a given position of a palm of a hand, a given position of a back of the hand, and a position of a wrist.

In the biological information measurement apparatus having this feature, measurement of a pulse wave signal is performed at any of an end of a finger, a base of the finger, a palm of a hand, a back of the hand, and a wrist. Thus, this biological information measurement apparatus can more reliably measure pulse wave signals.

Alternatively, in the above biological information measurement apparatus, the first measurement position and the second measurement position may be two positions of the living body selected from the group consisting of a position of an end of a toe, a position of a base of the toe, a given position of an instep, a given position of a sole, or a position of an ankle.

In the biological information measurement apparatus having this feature, in a situation where small fingers like newborn baby's fingers cause difficulty in measurement of a pulse wave signal, measurement of a pulse wave signal is performed at any of an end of a toe, a base of the toe, an instep, a sole, and an ankle. Thus, this biological information measurement apparatus can more reliably measure pulse wave signals.

Preferably, any one of the above biological information measurement apparatus further includes a cardiac output storage section configured to store therein the cardiac output calculated by the biological information calculation section, wherein the biological information calculation section is configured to further calculate, as the biological information, an amount of temporal change in the cardiac output, based on the cardiac output stored in the cardiac output storage section.

In the biological information measurement apparatus having this feature, the change amount of the cardiac output is calculated as the biological information. Thus, this biological information measurement apparatus can capture a temporal change in cardiac function. That is, this biological information measurement apparatus can capture a tendency toward deterioration in pumping ability of heart, and thus can find worsening of symptoms.

Preferably, in any one of the above biological information measurement apparatus, the biological information calculation section is configured to further calculate, as the biological information, a pulse wave velocity, based on the lag amount calculated by the time lag calculation section, and a distance between the first measurement position and the second measurement position.

Preferably, in any one of the above biological information measurement apparatus, the biological information calculation section is configured to further calculate, as the biological information, a blood vessel cross-sectional area or a relative blood pressure, based on the direct-current component calculated by the direct-current component calculation section.

The biological information measurement apparatus having these features can calculate the pulse wave velocity, the blood vessel cross-sectional area or the relative blood pressure, as the biological information other than the cardiac output.

Preferably, in any one of the above biological information measurement apparatus, the second pulse wave signal acquisition unit is configured to emit, as the second light beam, a light beam having a wavelength in a green wavelength band, to the living body, and receive a corresponding light beam reflected by the living body, to acquire the second pulse wave signal.

In the biological information measurement apparatus having this feature, by using a light beam having a wavelength in a green wavelength band, a pulse wave signal can be acquired from a corresponding light beam reflected by the living body. Thus, it becomes possible to acquire a pulse wave signal even in a region of the living body through which any light beam is not transmitted.

Preferably, any one of the above biological information measurement apparatus further includes a third pulse wave signal acquisition unit configured to emit a third light beam having a wavelength different from that of the first light beam, to the living body at the first measurement position, and receive a corresponding light beam transmitted through or reflected by the living body, to acquire a third pulse wave signal, wherein the biological information calculation section is configured to further calculate, as the biological information, an oxygen saturation of the living body, based on the first pulse wave signal and the third pulse wave signal.

The biological information measurement apparatus having this feature can calculate the oxygen saturation together with the cardiac output. In this case, the third pulse wave signal acquisition unit and the first pulse wave signal acquisition unit may be constructed such that they are contained in the same housing, or may be constructed such that they are contained, respectively, in separate housings.

Preferably, any one of the above biological information measurement apparatus further includes an inclination determination section configured to determine an inclination of the apparatus, wherein the biological information calculation section is configured to correct the cardiac output depending on the inclination determined by the inclination determination section.

In the biological information measurement apparatus having this feature, the cardiac output is corrected depending on the inclination of the biological information measurement apparatus, i.e., depending on a posture of a subject having the biological information measurement apparatus attached thereto, so that it becomes possible to more accurately calculate the cardiac output, irrespective of the posture of the subject.

Preferably, any one of the above biological information measurement apparatus further includes an inclination determination section configured to determine an inclination of the apparatus, wherein the biological information calculation section is configured to calculate the cardiac output only when the inclination determined by the inclination determination section falls within a predetermined range.

In the biological information measurement apparatus having this feature, the cardiac output is measured only when the biological information measurement apparatus has a given inclination, so that it becomes possible to measure the cardiac output in a situation where a subject having the biological information measurement apparatus attached thereto is always in the same posture. In the case where the biological information measurement apparatus is attached to a hand of a subject, examples of a posture of the subject include a posture where the hand is lifted above the heart, and a posture where the hand is lowered below the heart. That is, because an amount of blood flowing through blood vessels at the measurement position fluctuates depending on a posture of the subject, the cardiac output is measured under a condition that such a fluctuation is minimized. Thus, it becomes possible to increase reliability against temporal fluctuation of the calculated cardiac output.

Preferably, in any one of the above biological information measurement apparatus, the inclination determination section comprises a tri-axial acceleration sensor, wherein the inclination determination section is configured to determine an inclination of the apparatus, based on tri-axial gravitational acceleration components output from the tri-axial acceleration sensor, and wherein the biological information calculation section is configured to store the calculated biological information in the biological information storage section, in associated relation with the inclination determined by the inclination determination section.

In the biological information measurement apparatus having this feature, the biological information is stored in associated relation with the inclination of the apparatus, i.e., the posture of the subject during measurement, so that it becomes possible to know the biological information in the posture of the subject.

Preferably, any one of the above biological information measurement apparatus further includes a biological information storage section configured to store therein the biological information, wherein the biological information calculation section is configured to store a part or an entirety of the calculated biological information in the biological information storage section.

In the biological information measurement apparatus having this feature, the measured biological information is stored, so that it becomes possible to refer to past measurement results to observe a change in symptoms of the subject.

Preferably, any one of the above biological information measurement apparatus further includes a display unit, wherein the biological information calculation section is configured to display a part or an entirety of the calculated biological information on the display unit.

In the biological information measurement apparatus having this feature, biological information necessary for diagnosis can be selectively displayed to contribute to the diagnosis.

This application is based on Japanese Patent Application Serial No. 2013-208199 filed in Japan Patent Office on October 03, 2014, the contents of which are hereby incorporated by reference.

Although the present invention has been described appropriately and fully by way of the embodiment as above with reference to the drawings in order to express the present invention, it should be appreciated that anyone skilled in the art can readily change and/or modify the embodiment described above. It is therefore understood that a changed embodiment or a modified embodiment implemented by anyone skilled in the art is encompassed within the scope of the appended claims unless the changed embodiment or the modified embodiment is of a level that deviates from the scope of the appended claims.

Industrial Applicability

The present invention can provide a biological information measurement apparatus and a biological information measurement method.

**Claims**

1.   A biological information measurement apparatus comprising:

a first pulse wave signal acquisition unit configured to emit a first light beam to a first measurement position of a living body, and receive a corresponding light beam transmitted through or reflected by the living body, to acquire a first pulse wave signal;
a second pulse wave signal acquisition unit configured to emit a second light beam to a second measurement position of the living body different from the first measurement position, and receive a corresponding light beam transmitted through or reflected by the living body, to acquire a second pulse wave signal;
a time lag calculation section configured to calculate a temporal lag amount between a first time point at which the first pulse wave signal has a given phase, and a second time point at which the second pulse wave signal corresponding to the first pulse wave signal has the given phase;
a direct-current component calculation section configured to calculate a direct-current component, based on either one of the first pulse wave signal and the second pulse wave signal;
a pulse rate calculation section configured to calculate a pulse rate of the living body, base on either one of the first pulse wave signal and the second pulse wave signal; and
a biological information calculation section configured to calculate, as biological information, a cardiac output of the living body, based on the lag amount, the direct-current component and the pulse rate.

2.   The biological information measurement apparatus as recited in claim 1, wherein the first measurement position and the second measurement position are located on a pathway along which a pulse wave from a heart propagates, at respective different distances from the heart.

3.   The biological information measurement apparatus as recited in claim 2, wherein the first measurement position and the second measurement position are two positions of the living body selected from the group consisting of a position of an end of a finger, a position of a base of the finger, a given position of a palm of a hand, a given position of a back of the hand, and a position of a wrist.

4.   The biological information measurement apparatus as recited in claim 2, wherein the first measurement position and the second measurement position are two positions of the living body selected from the group consisting of a position of an end of a toe, a position of a base of the toe, a given position of an instep, a given position of a sole, or a position of an ankle.

5.   The biological information measurement apparatus as recited in any one of claims 1 to 4, which further comprises a cardiac output storage section configured to store therein the cardiac output calculated by the biological information calculation section, wherein
the biological information calculation section is configured to further calculate, as the biological information, an amount of temporal change in the cardiac output, based on the cardiac output stored in the cardiac output storage section.

6.   The biological information measurement apparatus as recited in any one of claims 1 to 5, wherein the biological information calculation section is configured to further calculate, as the biological information, a pulse wave velocity, based on the lag amount calculated by the time lag calculation section, and a distance between the first measurement position and the second measurement position.

7.   The biological information measurement apparatus as recited in any one of claims 1 to 6, wherein the biological information calculation section is configured to further calculate, as the biological information, a blood vessel cross-sectional area or a relative blood pressure, based on the direct-current component calculated by the direct-current component calculation section.

8. The biological information measurement apparatus as recited in any one of claims 1 to 7, wherein the second pulse wave signal acquisition unit is configured to emit, as the second light beam, a light beam having a wavelength in a green wavelength band, to the living body, and receive a corresponding light beam reflected by the living body, to acquire the second pulse wave signal.

9. The biological information measurement apparatus as recited in any one of claims 1 to 8, which further comprises a third pulse wave signal acquisition unit configured to emit a third light beam having a wavelength different from that of the first light beam, to the living body at the first measurement position, and receive a corresponding light beam transmitted through or reflected by the living body, to acquire a third pulse wave signal, wherein
the biological information calculation section is configured to further calculate, as the biological information, an oxygen saturation of the living body, based on the first pulse wave signal and the third pulse wave signal.

10. The biological information measurement apparatus as recited in any one of claims 1 to 9, which further comprises an inclination determination section configured to determine an inclination of the apparatus, wherein
the biological information calculation section is configured to correct the cardiac output depending on the inclination determined by the inclination determination section.

11. The biological information measurement apparatus as recited in any one of claims 1 to 10, which further comprises an inclination determination section configured to determine an inclination of the apparatus, wherein
the biological information calculation section is configured to calculate the cardiac output only when the inclination determined by the inclination determination section falls within a predetermined range.

12. The biological information measurement apparatus as recited in claim 10 or 11, wherein the inclination determination section comprises a tri-axial acceleration sensor, the inclination determination section being configured to determine an inclination of the apparatus, based on tri-axial gravitational acceleration components output from the tri-axial acceleration sensor, and wherein
the biological information calculation section is configured to store the calculated biological information in the biological information storage section, in associated relation with the inclination determined by the inclination determination section.

13. The biological information measurement apparatus as recited in any one of claims 1 to 12, which further comprises a biological information storage section configured to store therein the biological information, wherein
the biological information calculation section is configured to store a part or an entirety of the calculated biological information in the biological information storage section.

14. The biological information measurement apparatus as recited in any one of claims 1 to 12, which further comprises a display unit, wherein
the biological information calculation section is configured to display a part or an entirety of the calculated biological information on the display unit.

15. A biological information measurement method for use with a biological information measurement apparatus for, based on a first pulse wave signal and a second pulse wave signal obtained, respectively, from a first measurement position and a second measurement position which are different positions in a living body, to measure biological information of the living body, comprising:

a first pulse wave signal acquisition step of emitting a first light beam to the first measurement position, and receiving a corresponding light beam transmitted through or reflected by the living body, to acquire the first pulse wave signal;
a second pulse wave signal acquisition step of emitting a second light beam to the second measurement position, and receiving a corresponding light beam transmitted through or reflected by the living body, to acquire the second pulse wave signal;
a time lag calculation step of calculating a temporal lag amount between a first time point at which the first pulse wave signal has a given phase, and a second time point at which the second pulse wave signal corresponding to the first pulse wave signal has the given phase;
a direct-current component calculation step of calculating a direct-current component, based on either one of the first pulse wave signal and the second pulse wave signal;
a pulse rate calculation step of calculating a pulse rate of the living body, base on either one of the first pulse wave signal and the second pulse wave signal; and

a biological information calculation step of calculating, as biological information, a cardiac output of the living body, based on the lag amount, the direct-current component and the pulse rate.

FIG.1

EP 3 037 033 A1

FIG.2

# FIG.3

# FIG.4

# FIG.5

101

| DIRECT-CURRENT COMPONENT | k1 |
|---|---|
| ～dc1 | d11 |
| dc1～dc2 | d12 |
| dc2～dc3 | d13 |
| ... | ... |

# FIG.6

```
        ( CARDIAC OUTPUT )
        (  CALCULATION   )
        (   PROCESSING    )
                │
                ▼                          S11
        ┌──────────────────────┐
        │    ACQUISITION OF     │
        │ FIRST PULSE WAVE SIGNAL│
        └──────────────────────┘
                │
                ◇────────────────────────────────────────┐
                │                          │              │
                ▼          S12             │              │
        ┌──────────────────────┐          │              │
        │    ACQUISITION OF     │          │              │
        │ SECOND PULSE WAVE SIGNAL│        │              │
        └──────────────────────┘          │              │
 S13            │                          ▼   S15        ▼   S16
        ┌──────────────────────┐  ┌──────────────┐  ┌──────────────────┐
        │   CALCULATION OF      │  │ CALCULATION  │  │  CALCULATION OF   │
        │   INTER-PULSE-WAVE    │  │ OF PULSE RATE │  │  DIRECT-CURRENT   │
        │     LAG AMOUNT        │  │              │  │    COMPONENT      │
        └──────────────────────┘  └──────────────┘  └──────────────────┘
 S14            │                          │              │
        ┌──────────────────────┐          │              │
        │   CALCULATION OF      │          │              │
        │ PULSE WAVE VELOCITY   │          │              │
        └──────────────────────┘          │              │
                │◄─────────────────────────┴──────────────┘
                ▼          S17
        ┌──────────────────────┐
        │   CALCULATION OF      │
        │   CARDIAC OUTPUT      │
        └──────────────────────┘
                │          S18
        ┌──────────────────────┐
        │  STORAGE AND DISPLAY  │
        └──────────────────────┘
                │
                ▼
        ( TERMINATION OF )
        (  PROCESSING    )
```

# FIG.7

CARDIAC OUTPUT
CALCULATION
PROCESSING

↓

S11
ACQUISITION OF
FIRST PULSE WAVE SIGNAL

↓

S12
ACQUISITION OF
SECOND PULSE WAVE SIGNAL

↓

S13
CALCULATION OF
INTER-PULSE-WAVE
LAG AMOUNT

S15
CALCULATION
OF PULSE RATE

S16
CALCULATION OF
DIRECT-CURRENT
COMPONENT

↓

S14
CALCULATION OF
PULSE WAVE VELOCITY

S21
CALCULATION OF
BLOOD PRESSURE

↓

S22
CALCULATION OF
CARDIAC OUTPUT

↓

S18
STORAGE AND DISPLAY
(CARDIAC OUTPUT,
BLOOD PRESSURE)

↓

TERMINATION OF
PROCESSING

FIG.8

**200**

**30** MEASUREMENT CONTROL UNIT

**10** FIRST PULSE WAVE DETECTION UNIT
- **11** FIRST LIGHT-EMITTING SECTION
- **12** FIRST LIGHT-RECEIVING SECTION
- **13** THIRD LIGHT-EMITTING SECTION
- **14** THIRD LIGHT-RECEIVING SECTION

**31** LIGHT-EMITTING DRIVE SECTION
**32** I/V CONVERSION SECTION
**33** AMPLIFICATION SECTION
**34** A/D CONVERSION SECTION
**35** TIME LAG CALCULATION SECTION
**36** DIRECT-CURRENT COMPONENT CALCULATION SECTION
**37** PULSE RATE CALCULATION SECTION
**68** BIOLOGICAL INFORMATION CALCULATION SECTION (CARDIAC OUTPUT, $SpO_2$)
**39** BIOLOGICAL INFORMATION STORAGE SECTION

**20** SECOND PULSE WAVE DETECTION UNIT
- **21** SECOND LIGHT-EMITTING SECTION
- **22** SECOND LIGHT-RECEIVING SECTION

**40** INPUT UNIT

**50** DISPLAY UNIT

EP 3 037 033 A1

# FIG.9

```
        ┌──────────────────┐
        │  CARDIAC OUTPUT  │
        │   CALCULATION    │
        │   PROCESSING     │
        └──────────────────┘
                 │
                 ▼              ╭S11
        ┌──────────────────┐
        │  ACQUISITION OF  │
        │ FIRST PULSE WAVE SIGNAL │
        └──────────────────┘
                 │
                 ◇───────────────┬──────────────┬─────────────────┐
                 │               │              │                 │
                 ▼    ╭S12       │              │                 ▼        ╭S31
        ┌──────────────────┐     │              │        ┌──────────────────┐
        │  ACQUISITION OF  │     │              │        │ ACQUISITION OF THIRD │
        │ SECOND PULSE WAVE SIGNAL │            │        │  PULSE WAVE SIGNAL   │
        └──────────────────┘     │              │        └──────────────────┘
                 │               │              │                 │
                 │               │              │                 ▼        ╭S32
                 │               │              │        ┌──────────────────┐
                 │               │              │        │ CALCULATION OF SpO2 │
                 │               ▼   ╭S15       │        └──────────────────┘
                 │        ┌──────────────────┐  │                 │
                 │        │   CALCULATION    │  │                 │
                 │        │  OF PULSE RATE   │  │                 │
                 │        └──────────────────┘  │                 │
                 │               │              │                 │
        S13╮     │               │         ╭S16 │                 │
        ┌──────────────────┐     │        ┌──────────────────┐    │
        │  CALCULATION OF  │     │        │  CALCULATION OF  │    │
        │ INTER-PULSE-WAVE │     │        │  DIRECT-CURRENT  │    │
        │   LAG AMOUNT     │     │        │    COMPONENT     │    │
        └──────────────────┘     │        └──────────────────┘    │
        S14╮     │               │              │                 │
        ┌──────────────────┐     │              │                 │
        │  CALCULATION OF  │     │              │                 │
        │ PULSE WAVE VELOCITY │  │              │                 │
        └──────────────────┘     │              │                 │
                 │◄───────────────┴──────────────┘                 │
                 ▼   ╭S17                                          │
        ┌──────────────────┐                                      │
        │  CALCULATION OF  │                                      │
        │  CARDIAC OUTPUT  │                                      │
        └──────────────────┘                                      │
                 │◄──────────────────────────────────────────────┘
                 ▼   ╭S18
        ┌──────────────────┐
        │ STORAGE AND DISPLAY │
        │(CARDIAC OUTPUT,SpO2)│
        └──────────────────┘
                 │
                 ▼
        ┌──────────────────┐
        │ TERMINATION OF   │
        │  PROCESSING      │
        └──────────────────┘
```

# FIG.10

3900

| DATE-TIME | CARDIAC OUTPUT (ml/MINUTE) | $SpO_2(\%)$ | ... |
|---|---|---|---|
| 2013/06/07 08:30 | 100 | 94 | ... |
| 2013/07/08 09:05 | 87 | 92 | ... |
| ... | ... | ... | ... |
| 2013/09/06 18:25 | 115 | 90 | ... |
| ... | ... | ... | ... |

3901     3902     3903

FIG.11

300

**FIRST PULSE WAVE DETECTION UNIT** 10
- FIRST LIGHT-EMITTING SECTION 11
- FIRST LIGHT-RECEIVING SECTION 12

**SECOND PULSE WAVE DETECTION UNIT** 20
- SECOND LIGHT-EMITTING SECTION 21
- SECOND LIGHT-RECEIVING SECTION 22

**MEASUREMENT CONTROL UNIT** 30
- LIGHT-EMITTING DRIVE SECTION 31
- I/V CONVERSION SECTION 32
- AMPLIFICATION SECTION 33
- A/D CONVERSION SECTION 34
- TIME LAG CALCULATION SECTION 35
- DIRECT-CURRENT COMPONENT CALCULATION SECTION 36
- PULSE RATE CALCULATION SECTION 37
- BIOLOGICAL INFORMATION CALCULATION SECTION (CARDIAC OUTPUT) 78
- BIOLOGICAL INFORMATION STORAGE SECTION 79

- ACCELERATION SENSOR 71
- POSTURE DETERMINATION SECTION 72
- DETERMINATION CONDITION STORAGE SECTION 73

INPUT UNIT 40

DISPLAY UNIT 50

EP 3 037 033 A1

# FIG.12

7300

| X VECTOR | Y VECTOR | Z VECTOR | POSTURE |
|----------|----------|----------|---------|
| $x0 \sim x1$ | $y0 \sim y1$ | $z0 \sim z1$ | UPWARD |
| $x2 \sim x3$ | $y2 \sim y3$ | $z2 \sim z3$ | HORIZONTAL |
| $x4 \sim x5$ | $y4 \sim y5$ | $z4 \sim z5$ | DOWNWARD |

7301 7302 7303 7304

FIG.13A

FIG.13B

FIG.13C

# FIG.14

```
        ╭─────────────────╮
        │ CARDIAC OUTPUT  │
        │  CALCULATION    │
        │  PROCESSING     │
        ╰─────────────────╯
                 │
                 ▼            S41
        ┌─────────────────┐
        │  DETERMINATION  │
        │   OF POSTURE    │
        └─────────────────┘
                 │
                 ▼            S11
        ┌─────────────────┐
        │  ACQUISITION OF │
        │FIRST PULSE WAVE SIGNAL│
        └─────────────────┘
                 │
                 ◇──────────────────────●────────────────────┐
                 │                       │                    │
                 ▼            S12        │                    │
        ┌─────────────────┐            │                    │
        │  ACQUISITION OF │            │                    │
        │SECOND PULSE WAVE SIGNAL│      │                    │
        └─────────────────┘            │                    │
 S13             │              S15     ▼           S16      ▼
   ┌─────────────────┐   ┌─────────────────┐   ┌─────────────────┐
   │  CALCULATION OF │   │  CALCULATION    │   │  CALCULATION OF │
   │ INTER-PULSE-WAVE│   │  OF PULSE RATE  │   │ DIRECT-CURRENT  │
   │   LAG AMOUNT    │   │                 │   │   COMPONENT     │
   └─────────────────┘   └─────────────────┘   └─────────────────┘
 S14             │                │                      │       S42
   ┌─────────────────┐            │             ┌─────────────────┐
   │  CALCULATION OF │            │             │   CORRECTION    │
   │ PULSE WAVE VELOCITY│         │             │   DEPENDING     │
   └─────────────────┘            │             │   ON POSTURE    │
                 │                │             └─────────────────┘
                 └────────────────┴──────────────────────┘
                 │
                 ▼            S17
        ┌─────────────────┐
        │  CALCULATION OF │
        │  CARDIAC OUTPUT │
        └─────────────────┘
                 │
                 ▼            S18
        ┌─────────────────┐
        │STORAGE AND DISPLAY│
        └─────────────────┘
                 │
                 ▼
        ╭─────────────────╮
        │ TERMINATION OF  │
        │   PROCESSING    │
        ╰─────────────────╯
```

33

# FIG.15

7900

| DATE-TIME | CARDIAC OUTPUT (ml/MINUTE) | $SpO_2$(%) | · · · | POSTURE |
|---|---|---|---|---|
| 2013/06/07 08:30 | 100 | 94 | · · · | UPWARD |
| 2013/07/08 09:05 | 87 | 92 | · · · | HORIZONTAL |
| · · · | · · · | · · · | · · · | · · · |
| 2013/09/06 18:25 | 115 | 90 | · · · | HORIZONTAL |
| · · · | · · · | · · · | · · · | · · · |

7901    7902    7903    7904

## INTERNATIONAL SEARCH REPORT

| International application No. |
|---|
| PCT/JP2014/073977 |

**A. CLASSIFICATION OF SUBJECT MATTER**
*A61B5/026*(2006.01)i, *A61B5/0205*(2006.01)i, *A61B5/0245*(2006.01)i

According to International Patent Classification (IPC) or to both national classification and IPC

**B. FIELDS SEARCHED**

Minimum documentation searched (classification system followed by classification symbols)
A61B5/026, A61B5/0205, A61B5/0245

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2011-239972 A (Seiko Epson Corp.), 01 December 2011 (01.12.2011), entire text; all drawings & CN 102247169 A | 1-15 |
| A | JP 2001-321347 A (Nihon Kohden Corp.), 20 November 2001 (20.11.2001), entire text; all drawings & US 2002/0002339 A1 | 1-15 |
| A | WO 2002/085204 A1 (Combi Corp.), 31 October 2002 (31.10.2002), entire text; all drawings & TW 529931 B & CN 1461201 A | 1-15 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered    to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 02 December, 2014 (02.12.14) | 16 December, 2014 (16.12.14) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2014/073977

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2013-70732 A  (Toshiba Corp.),<br>22 April 2013 (22.04.2013),<br>entire text; all drawings<br>& US 2013/0079648 A1 | 1-15 |
| A | JP 2002-224063 A  (Aloka Co., Ltd.),<br>13 August 2002 (13.08.2002),<br>entire text; all drawings<br>(Family: none) | 1-15 |
| A | JP 2009-543609 A  (Edwards Lifesciences Corp.),<br>10 December 2009 (10.12.2009),<br>entire text; all drawings<br>& US 2008/0015451 A1     & EP 2053964 A2<br>& WO 2008/019207 A2     & CA 2656815 A1<br>& CN 101489472 A1        & AU 2007281884 A1 | 1-15 |
| A | JP 2010-246801 A  (Nihon Kohden Corp.),<br>04 November 2010 (04.11.2010),<br>entire text; all drawings<br>& US 2010/0268101 A1     & EP 2241251 A1 | 1-15 |
| A | CN 101176663 A  (Shandong University),<br>14 May 2008 (14.05.2008),<br>entire text; all drawings<br>(Family: none) | 1-15 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005312947 A
- JP 4613261 B
- JP 2013208199 A